# EUROPEAN PATENT APPLICATION

(11) **EP 4 613 852 A1**
(43) Date of publication of application: **10.09.2025**
(21) Application number: 23881348.9
(22) Date of filing: 23.07.2023
(51) Int. Cl.: C12N 5/078, C12N 5/10, A61K 35/18, A61K 39/00, A61P 35/00, A61P 37/04

(54) **METHOD FOR COUPLING THERAPEUTIC MOLECULES TO SURFACES OF MATURE ERYTHROCYTES AND USE**

(30) Priority: 26.10.2022 CN 202211315211
(71) Applicant: Ray Medicine Biotechnology Co., Ltd, Beijing 100089 (CN)
(72) Inventor: WANG, Jishu, Beijing 100089 (CN); HU, Tanyu, Beijing 100089 (CN); HUANG, Tao, Beijing 100089 (CN)
(74) Representative: dompatent von Kreisler Selting Werner - Partnerschaft von Patent- und Rechtsanwälten mbB
(86) International application number: PCT/CN2023/108773
(87) International publication number: WO 2024/087760

(57) **Abstract**

Disclosed in the present invention is a method for coupling mature erythrocytes *in vitro. A* drug (including macromolecules such as antibodies, polypeptides and nucleotides, and chemical molecules such as chemotherapeutic drugs) is coupled to glycoproteins on the surfaces of erythrocyte membranes by means of glycosidic bonds, thereby reserving the characteristics of integrity, deformation capacity, oxygen carrying capacity and long half-life of mature erythrocyte membranes, and reserving the biological activity of the drug. The method is used for treating tumors, metabolic diseases and inflammatory diseases.

## Description

### TECHNICAL FIELD

The disclosure discloses a method for preparing engineered cells, which belongs to the technical field of cell engineering.

### BACKGROUND TECHNOLOGY

CD8⁺ T cells and NK cells are cytotoxic effector cells in the immune system, which can recognize and kill infected or malignant cells. CD8⁺ T cells and NK cells can be stimulated by various strategies for the purpose of eliminating or reducing cancer cells in clinical cancer therapy. There are complicated and confused results in clinical trials, and the unsatisfactory therapeutic effect is not only related to the type of cancer and administration manners, but also attributed to the short half-life and potential toxicity from the effect on non-immune cells [2019-Star Review The challenges and molecular approaches surrounding interleukin-2-based; 2019-IL 15 for cancer and autoimmune; 2021-Next-generation cytokines for cancer immunotherapy; 2020-Cancer-killing, decoy-resistant interleukin-18] . Furthermore, immuno-co-stimulating molecule, being the second signal for stimulating immune cell, is coming into the clinical trial phase, including CD27, CD40, OX40, GITR, ICOS, etc. For example, 4-1BB (CD137, tumor necrosis factor ligand superfamily member 9) belongs to the tumor necrosis factor receptor superfamily, and is mainly expressed on the surface of various immune cells, including activated T lymphocyte (CD4⁺ and CD8⁺), NK, macrophage, B lymphocyte, etc. Pre-clinical study shows, the activation by 4-1BB signal stimulates the cytotoxic T lymphocyte, and NK cell, and the effects cause continuous immunologic memory reaction. However, due to the diffuse expression of 4-1BB, the activator for 4-1BB is easy to cause a systemic adverse reaction. After two deaths related with hepatotoxicity occurred in December 2008 when Urelumab was the activator for 4-1BB, all the clinical studies of Urelumab were stopped. Afterwards, a detailed analysis on clinical safety data shows, that dose of Urelumab is the single most important factor contributing to severe hepatic damage [2017-Results from an Integrated Safety Analysis of Urelumab, an Agonist Anti-CD137 Monoclonal Antibody] . Further study shows, Urelumab entered into liver parenchyma through blood circulation, then activated the hepatic-inherent macrophages, and caused non-specific damage to hepatic cells [2018-Activation of 4-1BB on liver myeloid cells triggers hepatitis] .

Chimeric Antigen Receptor T-Cell Immunotherapy, Chimeric Antigen Receptor T-Cell Immunotherapy (CAR-T), is a new engineered T cell adoptive treatment. It can recognize cancer cells, activate engineered T cells and kill the tumor, but the technical process of CAR-T is complicated and expensive. The main challenge in cell engineering such as CAR-T is providing new properties to the engineered cells without destroying the endogenous function of the cells. As the most common and robust cell engineering method at present, the method is first limited by technical complexity and safety concerns, for example, inconsistent reproducibility of viral transduction efficiency of primary cells, heterogeneous expression levels of CAR genes, and possibility of endogenous gene destruction.

Direct modification on the cell surface by chemical biological tools has become a supplemental and common method. Glycosylation is a process of covalently linking carbohydrates and target molecules (usually proteins and lipids). Protein glycosylation, being an enzymatic reaction in the absence of a template, is carried out by a donor molecule, usually an activated nucleotide sugar, targeting the site (hydroxyl or other functional groups) of a receptor and conducing a specific glycoconjugate reaction under the action of glycosyltransferases. Fucose, as a constituent of sugar chain in glycoproteins, is present widely on the plasma membrane of various types of cell surfaces. Fucosyltransferase is an enzyme that transfers L-fucose from GDP-fucose (guanosine diphosphate fucose) donor substrates to receptor substrates. According to reports in various current literature, the main donor substrates of fucosyltransferase are the GDP-fucose of a relatively small molecular weight. Fucosyltransferase catalyzes the transfer of fucoside to the N-polysaccharide of mammalian glycoprotein. Wu, et al., by using fucosyltransferase of Helicobacter pylori, successfully transferred macromolecular protein such as antibody to polysaccharides such as LacNAc and α2,3 sialyl LacNAc on the cell membrane surface. Wu, et al. constructed two kinds of engineered cells by this method - using natural killer cell line (NK-92MI) and mouse primary CD8⁺OT-1T cells and transferring antibody to Her2 and antibody to PD-L1 to NK-92MI cells and CD8⁺OT-1T cells respectively, by fucosyltransferase, which demonstrated specific tumor targeting and inhibitory signals against tumor cells in a mouse model (See Li J, et al. ACS Cent Sci. 2018 Dec 26; 4 (12): 1633-1641.). Thus, the method, which tags the target molecule coupled with the donor substrate onto the target cell coupled with the receptor substrate by fucosyltransferase, will significantly improve the effectiveness of cell therapy such as CAR-T. Although being a beneficial supplement to the CAR-T and immune cell adoptive therapy, this method is still a highly customized therapy, which is limited by the quantity of available patient immune cells, complexity of cell amplification *in vitro,* high operation cost, long operation time, and the difficulty in sorting immune cell subtypes, resulting in high prices. Additionally, immune cells have a short half-life *in vivo,* and are easily inhibited by the tumor microenvironment, resulting in functional exhaustion, which limits its practical application.

Due to the above-mentioned technical shortcomings in the prior art, the objective of this disclosure is to provide a new method for tumor immunotherapy, to activate existing NK cells and CD8⁺ T cells *in vivo* directly or indirectly, effectively, and highly specifically, so as to be different from the highly customized cell therapy in the prior art (it is necessary to amplify *in vitro* to increase the quantity of NK and T cells , and then transfer them back to the patient).

### SUMMARY OF THE DISCLOSURE

Based on the above-mentioned objective, this disclosure first provides a method that couples chemical molecules or biomacromolecules on the membrane of mature red blood cells, said method comprising the following steps:
(1) Modifying the chemical molecules or biomacromolecules by covalently coupling a GDP-fucose derivative to the chemical molecules or biomacromolecules.
(2) Covalently coupling the chemical molecules or biomacromolecules obtained from step (1) with a polysaccharide on a membrane surface of a red blood cell by a glycosylic bond usin an enzymatic reaction mediated by fucosyltransferase.

Said GDP-fucose derivatives in this disclosure, such as GDP-fucose-(dibenzocyclo)-triazole-PEGn (n=0-12) and GDP-fucose-Lactic Acid-Glycolic Acid, all can realize the coupling with the chemical molecules or biomacromolecules. In an embodiment of this disclosure, said GDP-fucose derivative is Guanosine 5'- diphosphate-fucose-triazole-polyethylene glycol-methyl tetrazine (GDP-Fucose-Triazole-PEG4-Tz).

In a preferred embodiment, the chemical molecule is selected from therapeutic molecules or fluorophores. Small therapeutic molecules include but not limited to corticosteroids such as dexamethasone, anticoagulant drugs such as warfarin, platelet aggregation inhibiting drugs such as clopidogrel or ticagrelor, angiotensin system inhibitors such as captopril, and antianginal drugs such as trimetazidine; the fluorophores include but not limited to Cy5, Cy3.

In another preferred embodiment, the biomacromolecule is selected from polynucleotide, polypeptide or antibody. The polynucleotide includes but not limited to a CpG-containing single stranded oligonucleotide TCCATGACGTTCCTGACGTT, TCGTCGTTTTGTCGTTTTGTCGTT, CCTGGATGGGAACTTACCGCTGCA; the polypeptide includes but not limited to vascular endothelial somatostatin, glutathione, somatostatin, glatiramer acetate and Fc-fusion protein; the antibody includes but not limited to anti-VEGF antibody, anti-4-1BB antibody, anti-Tie2 antibody, anti-CD40 antibody.

More preferred, said antibody is selected from a monoclonal antibody, a single chain antibody, a bi-specific antibody, a nano antibody.

Particularly preferred, said antibody is anti-4-1BB activating antibody.

In another preferred embodiment, the biomacromolecule is selected from interleukin IL-15 isomer or interleukin IL-12.

In another preferred embodiment, the mature red blood cell is obtained from peripheral blood without serum and other blood cells, red blood cell suspension without free protein molecules, fresh red blood cell suspension or red blood cell suspension stored in suitable conditions within 30 days.

In another preferred embodiment, said fucosyltransferase is from a human being or from Helicobacter pylori.

In a more preferred embodiment, said GDP-fucose derivative is Guanosin 5'- diphosphate-fucose-triazole-polyethylene glycol-methyl tetrazine.

More preferred, the reaction condition of the enzymatic reaction mediated by fucosyltransferase in said Step (2) is: 5×10⁷/mL - 5×10⁹/mL for red blood cell density; 40 µg/mL - 300 µg/mL of fucosyltransferase; 60 µg/mL - 120 µg/mL of antibody or Fc fusion protein as the macromolecular drug; 4°C-37°C for reaction temperature; 20 minutes-16 hours of reaction time.

In a specific embodiment of this disclosure, when the red blood cell density is 5×10⁷/mL, an optimal coupling effect with human IL-15-Fc fusion protein derivative (also referred to as N803 in the present disclosure) can be achieved.

Especially preferred, said enzymatic reaction mediated by fucosyltransferase is carried out without Mg²⁺ in a reaction system of 150mM NaCl, 2.7mM KCl, 44mM glucose, pH 5.9-6.3. In recent reports [Jie, L. , et al. "A Single-Step Chemoenzymatic Reaction for the Construction of Antibody-Cell Conjugates." ACS Central Science 4.12(2018).] , fucosyltransferase is Mg²⁺ - dependent, and 20 mM of Mg²⁺ is necessary in an enzymatic reaction by fucosyltransferase. However, in pharmaceutical field, during the process of drug manufacture, any additive may affect the quality and safety of the final drug, especially the latter. Changes in metal ion concentration can cause various organ dysfunction, such as hypermagnesemia, which is shown as loss of appetite, nausea, vomiting, skin flushing, headache, dizziness in early stage, which may be easily neglected due to non-specificity. When the serum magnesium concentration reaches 2-4 mM, obvious changes can occur in neuromuscular and circulatory systems. Furthermore, the presence of Mg²⁺ can affect the integrity and half-life of red blood cells. The main components of the commercial red blood cell preservation solution are sodium chloride, sodium citrate, citric acid, and glucose. At 4°C, red blood cells can be preserved for 2 weeks without change in activity and specificity. They are often used for blood collection, preservation, and transportation of red blood cells and do not contain Mg²⁺. (Ye Hanquan et al., Dynamic Observation of Quality Control of Red Blood Cells Suspended and Washed in Red Blood Cell Preservation Fluid, Journal of Yangtze University (Natural Science Edition) Medical Journal, 2013). Experimental studies showed that, when 20 mM of Mg²⁺ is added into the PBS buffer or red blood cell preservation solution, even at 4°C, red blood cells will still rupture and hemolyze (data not shown). Therefore, removing additives and excipients as much as possible during the manufacturing process can minimize safety risks of drug use to patients to the greatest extent possible.

In the present disclosure, a macromolecular drug coupled with a GDP-Fucose derivative, such as an antibody or fusion protein coupled with GDP-Fucose-Triazole-PEG4-Tz (Guanosine 5'-diphosphate-fucose-triazole-polyethylene glycol-methyl tetrazine) (abbreviated as GDP-Fucose-antibody), is a macromolecular substrate (antibody or fusion protein, MW >50 kD), not a small molecular substrate of fucosyltransferase (such as the commercial derivative, GDP- Fucose-Azido, MW of only 630 Da). Another substrate of fucosyltransferase is a polysaccharide on cell membrane glycoproteins such as LacNAc. Under the action of fucosyltransferase, GDP-Fucose-antibody forms a glycosidic bond with LacNAc and releases GDP, forming (cell membrane) LacNAc-fucose-antibody, thereby coupling the antibody molecule onto the cell membrane. From the analysis on the molecular structure, the steric hindrance between fucosyltransferase and the GDP-Fucose-antibody is significantly greater than that between fucosyltransferase and a small molecule substrate. The steric hindrance also affects the action of Mg²⁺, thereby affecting the effect of Mg²⁺ on enzymatic activity.

The present disclosure adequately considers the above-mentioned adverse effects of Mg²⁺ on red blood cells and the steric hindrance of fucosyltransferase substrates on enzymatic reactions, as well as the fact that free phosphate molecules can act as products to inhibit enzymatic reactions. By removing free phosphate molecules from the reaction system, the adverse factors caused by the lack of Mg²⁺ can be offset. Specifically, the conventional PBS buffer solution (137mM sodium chloride, 10mM phosphate, 2.7mM potassium chloride; pH 7.4) was adjusted to a phosphate-free equilibrium solution named as NPBS (Non-Phosphate Buffer Solution): 150mM sodium chloride, 2.7mM potassium chloride, 44mM glucose), and the pH was adjusted from 7.2-7.6 of a conventional environment to 5.9-6.3 of an acidic environment. The adjusted reaction system is more in line with the *in vitro* survival conditions of red blood cells. The results showed that Mg²⁺ can be removed from the reaction system without affecting the enzymatic reaction.

Further, the present disclosure provides an engineered red blood cell, which is prepared according to the above-mentioned method.

Finally, the present disclosure provides a use of the above-mentioned engineered red blood cell in the preparation of therapeutic drugs for treating tumor diseases.

In a preferred embodiment, the tumor disease is colon cancer cells or melanoma.

Excellent technical effects of the present disclosure are mainly shown as follows:

The present disclosure couples one or more exogenous stimulatory molecules (such as 4-1BBL or a combination thereof) capable of activating and/or amplifying NK cells and/or CD8⁺ T cells on the surface of mature red blood cells in peripheral blood. The engineered red blood cells are used to stimulate circulating immune cells (such as NK cells and/or CD8⁺ T cells) in peripheral blood. The present disclosure has found that the engineered red blood cells containing IL-12, IL-15/IL-15RA, 4-1BBL, or a combination thereof can effectively activate primary CD4⁺, CD8⁺, NK cells and induce cytotoxicity.

The engineered red blood cells provided by the present disclosure have intact red blood cell membranes due to the fact that the coupling sites do not directly involve amino acid molecules on the membrane surface of the cell, and the coupling reaction is rapid and the conditions are mild. This is shown as no significant difference in deformability of the red blood cell membranes, intracellular 2,3-DPG, and free hemoglobin between the engineered red blood cells and natural red blood cells. The immunostimulatory molecules coupled to the surface of the red blood cells can maintain stimulation signals in the circulatory system with a long half-life, thus providing a safer and more effective method of stimulating immune killer cells.

The red blood cells provided by the present disclosure have been transformed into engineered erythroid cells. The engineered erythroid cells can be nucleated, such as erythroid precursor cells (such as red blood cell precursor cells), or they can be enucleated erythroid cells, such as reticulocytes or red blood cells. The present disclosure also provides the use of these engineered erythroid cells in activating NK cells and/or CD8⁺ T cells in a subject, such as a cancer or infectious disease subject, in need of them. The engineered red blood cells, including IL-12, IL-15/IL-15RA, 4-1BBL, and their combinations, can effectively slow down tumor growth and reduce tumor burden *in vivo.* In addition, the drugs prepared by the method of the present disclosure are off-the-shelf products, which have natural advantages in terms of convenience, accessibility, and production cost.

### BRIEF DESCRIPTION OF THE DRAWINGS

Fig. 1. Diagram of coupling a therapeutic molecule to mature red blood cell surface and its application;
Fig. 2. Analysis by mass spectrometry on molecular weight of N803 before and after coupling;
Fig. 3. Histogram of N803 protein quantity on red blood cell membrane detected by flow cytometry;
Fig. 4. Flow cytometry analysis on uncoupled red blood cells and red blood cells coupled with N803 protein;
Fig. 5. Flow cytometry analysis on the influence of red blood cell density on the coupling of antibodies;
Fig. 6. Flow cytometry analysis on the influence of fucosyltransferase concentration on the coupling of antibodies;
Fig. 7. Flow cytometry analysis on the influence of N803 protein on the coupling of antibodies (Part 1);
Fig.8. Flow cytometry analysis on the influence of N803 protein on the coupling of antibodies (Part 2);
Fig. 9. Flow cytometry analysis on the influence of N803 protein on the coupling of antibodies (Part 3);
Fig. 10. Flow cytometry analysis on the influence of temperature on the coupling of N803 protein with red blood cells;
Fig. 11 Flow cytometry analysis on the influence of Mg²⁺ on the coupling of 3H3 protein with HEK293 cell membrane;
Fig. 12. Histogram of 3H3 protein quantity on red blood cell membrane detected by flow cytometry;
Fig. 13. Histogram of Avastin protein quantity on red blood cell membrane detected by flow cytometry;
Fig. 14. Analysis on ATP content in mouse red blood cell after being coupled with protein;
Fig. 15. Analysis on the influence of incubation time on ATP content in mouse red blood cells;
Fig. 16. Analysis on the influence of protein concentration on ATP content in mouse red blood cells;
Fig. 17. Analysis on the influence of fucosyltransferase concentration on ATP content in mouse red blood cells;
Fig. 18. Analysis on the influence of coupled antibodies on the 2,3-DPG content of red blood cells;
Fig. 19. Analysis on the influence of coupled antibodies on the integrity of red blood cell membrane;
Fig. 20. Analysis on the influence of incubation time on the integrity of red blood cell membrane;
Fig. 21. Analysis on the influence of protein concentration on the integrity of red blood cell membrane;
Fig. 22. Analysis on the influence of fucosyltransferase concentration on the integrity of red blood cell membrane;
Fig. 23. Analysis on the influence of two coupling methods on the deformability of red blood cells;
Fig. 24. Analysis of the influence of two coupling methods on red blood cell homogeneity;
Fig. 25. Evaluation on half-life of mouse red blood cells *in vivo* coupled with antibodies;
Fig. 26. Diagram of *in vitro* detection of 4-1BB activation by the reporter gene system;
Fig. 27. Analysis on the activation ability of mouse red blood cells coupled with 3H3 antibody on the reporter genes;
Fig. 28. Analysis on CD8⁺ T cells and NK cells generated by co-stimulation of red blood cells coupled with 3H3;
Fig. 29. Analysis on the influence of mouse red blood cells coupled with anti-4-1BB antibody on CD8⁺ T cells secreting interferon-γ;
Fig. 30. Trend map of tumor volume changes after infusion of red blood cells coupled with antibody 3H3.

### SPECIFIC EMBODIMENTS

The following will further describe the present disclosure in conjunction with specific embodiments, and the advantages and features of the present disclosure will become clearer as described. However, these embodiments are only exemplary and do not constitute any limitation on the scope of protection defined by the claims of the present disclosure.

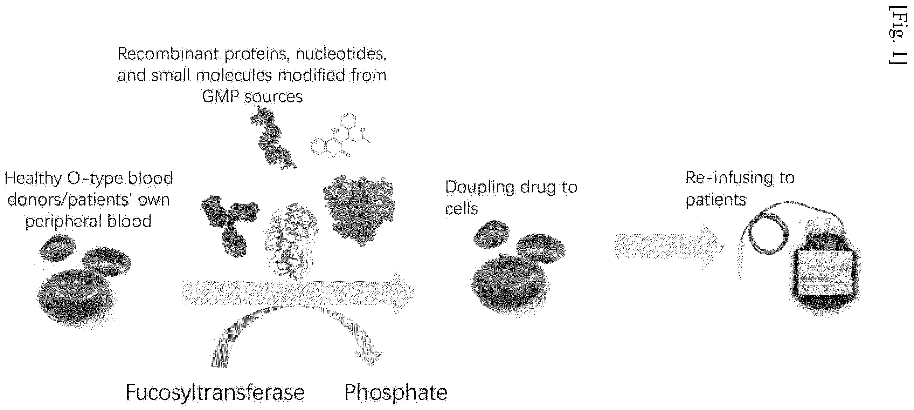

Figure 1 shows a flowchart of the method of the present disclosure for coupling chemical molecules or biomacromolecules with GDP fucose derivatives onto the surface of mature red blood cells through fucosyltransferase and then reintroducing them into a patient's body. The following is a description of specific implementation methods involved in the afore-mentioned flowchart.

### Example 1. Recovery of red blood cells from Balb/C mice and humans

Select 10-12 week old female Balb/C mice with SPF grade, disinfect under anesthesia, draw heart blood, transfer to K2-EDTA coated centrifuge tubes to prevent coagulation, store at 4°C. 0.5-0.8 mL whole blood could be obtained from each mouse. Centrifuge at 500 × g under 4°C for 5 minutes, and mark the levels of hematocrit (red, lower layer) and plasma (yellow, upper layer) on a test tube. Slowly and completely extract blood plasma and intermediate sediment (buffy coat) using a micropipette, add bleach to the extracted liquid, and discard it into biohazardous waste. Transfer 0.1 mL of red blood cell hematocrit to a 1.5 mL centrifuge tube, add 1 mL of PBS pH 7.4 solution and cover with a lid. Invert several times and mix well. Centrifuge at 500×g under 4 °C for 5 minutes, extract the supernatant and discard it. Repeat the PBS washing step 3-4 times. Add 100 µL -1000 µL PBS to the red blood cells after washing. Store at 4 °C for later use.

Collect 2-3 mL blood from anterior cubital vein of anonymous healthy blood donors. After disinfecting the skin, a disposable vacuum blood collection device was operated by professionals. Let the blood slowly flow along the wall of the test tube. When the blood reaches the marked area, pull out the tube, quickly invert several times, and mix evenly to prevent coagulation. Centrifuge at 500 × g under 4 °C for 5 minutes, and mark the levels of hematocrit (red, lower layer) and plasma (yellow, upper layer) on the test tube. Slowly and completely extract blood plasma and intermediate sediment (buffy coat) using a micropipette, add bleach to the extracted liquid, and discard it into biohazardous waste. Transfer 0.5 mL of red blood cell hematocrit to a 15 mL centrifuge tube, add 5 mL of PBS pH 7.4 solution and cover with a lid. Invert several times and mix well. Centrifuge at 500×g under 4 °C for 5 minutes, extract the supernatant and discard it. Repeat the PBS washing step 3-4 times. Add 1000 µL PBS to the red blood cells after washing. Store at 4 ° C for later use.

### Example 2. Coupling of GDP fucose with N803 and mass spectrometry detection

N803 (also known as ALT803) is a Fc fusion protein derivative with human IL-15 [2013

The IL 15 based superagonist ALT 803 promotes the antigen independent conversion of memory CD8 T cells into innate like effector cells with antitumor]. Based on the sequences published in the literature, corresponding DNA is designed and fused with the human IgG4-Fc sequence. Suzhou Junji Biotechnology Co., Ltd. was commissioned to synthesize plasmid DNA and clone it into vector pRM293 (PRM293 was obtained by modifying the plasmid pTT5, as detailed below: Shi C. Purification and characterization of a recombinant G-protein-coupled receptor, Saccharomyces cerevisiae Ste2p, transiently expressed in HEK293 EBNA1 cells. Biochemistry. 2005;44(48):15705-15714.). Suzhou Junji Biotechnology Co., Ltd. was commissioned to transiently transfect the plasmids into HEK293 cells (National Research Council, Canada) and perform affinity purification using Mabselect sure (Protein A, GE Healthcare). Couple GDP fucose to purified N803 by crosslinking the amine with N-hydroxysuccinimide ester (NHS ester) for later use. Unless otherwise stated, N803 means a GDP-fucose conjugated N803.
1. Crosslinking agents activated by NHS ester and labeled compounds react with the primary amine on N803 under physiological to weakly alkaline conditions (pH 7.2 to 9) to form a stable amide bond. Under this principle, the NHS on TCO-PEG4-NHS reacts with the primary amine on the N803 protein to form TCO-PEG4-N803. The specific methods and processes are as follows:
   React 400 µg of purified N803 protein with 150 µg of TCO-PEG4-NHS (Shanghai Premedic Pharmaceutical Technology Co., Ltd., Cat# A34125) and add 20 mM HEPES buffer (Thermofisher Scientific (China) Co., Ltd., Cat# 15630). The reaction product is incubated at room temperature for 30 minutes, 5 µmol of Tris buffer is added to terminate the reaction. Then the reaction is incubated at room temperature for 5 minutes, and the reaction product is loaded onto a PD SpinTrap G-25 desalination column (Cytiva, Cat# 28918004) and centrifuged at 800 × g to remove unreacted and small molecules formed from the reaction, to obtain the pure TCO-PEG4-NHS.
2. Based on the reverse electron demand in the Diels Alder cycloaddition reaction between trans cyclooctene and tetrazine, the connection between TCO (trans cyclooctene) and Tz (Tetrazine) forms a dihydropyridazine bond. Under this principle, TCO-PEG4-N803 reacts with the derivative of GDP fucose, GDP-Fucose-Triazole-PEG4-Tz, and the GDP-Fucose-Triazole-PEG4-PEG4-N803, i.e. GNP-fucose coupled N803, is obtained. The procedures are as follows:
   Further react the TCO-PEG4-N803 obtained in the previous step with 30 µg of GDP-Fucose-Triazole-PEG4-Tz (synthesized by Yantang Biotechnology Co., Ltd., Cat# YT-HJP-3-29). After incubation at room temperature for 30 minutes, load the reaction product onto a PD SpinTrap G-25 desalination column (Cytiva company, Cat# 28918004). Centrifuge at 800 × g to remove unreacted and small molecules formed from the reaction, and pure GDP-fucose coupled N803 is obtained.
3. MALDI-TOF determination of molecular weight of N803 protein before and after coupling with GDP fucose derivatives

The coupling of GDP fucose derivatives to lysine in the protein molecules is achieved by crosslinking N-hydroxysuccinimide ester (NHS ester) with amines. After coupling with each lysine, the molecular weight increases by approximately 1462.5Da. By comparing the relative molecular weight of the protein molecules before and after the coupling, the number of GDP fucose derivatives coupled with the proteins can be calculated. Soft ionization mass spectrometry technology-matrix assisted laser desorption ionization time-of-flight mass spectrometry (MALDI-TOF) can determine the relative molecular weight of glycoproteins, peptides, and amino acids. This example accurately obtained the molecular weight of N803 protein before and after being coupled with the GDP fucose derivatives through MALDI-TOF. The mass spectrometer G2-XSQ Tof/Tof (Waters Corporation, US) was operated by Shanghai Iproteome Biotechnology Co., Ltd. The experimental method is as follows:
1) Sampling: Place 0.5 µL of the sample onto the sample target, let it dry naturally, then spot 0.5 µL of SA matrix solution onto the corresponding target site and let it dry naturally;
2) Calibration: Select a linear method to calibrate the sample detection range in a positive ion mode;
3) Testing sample: Select a linear method to test the molecular weight of the sample in the positive ion mode;
4) Mass spectrometry data and spectrum processing: The raw data and spectra generated by MALDI-TOF are processed by the instrument's built-in software.

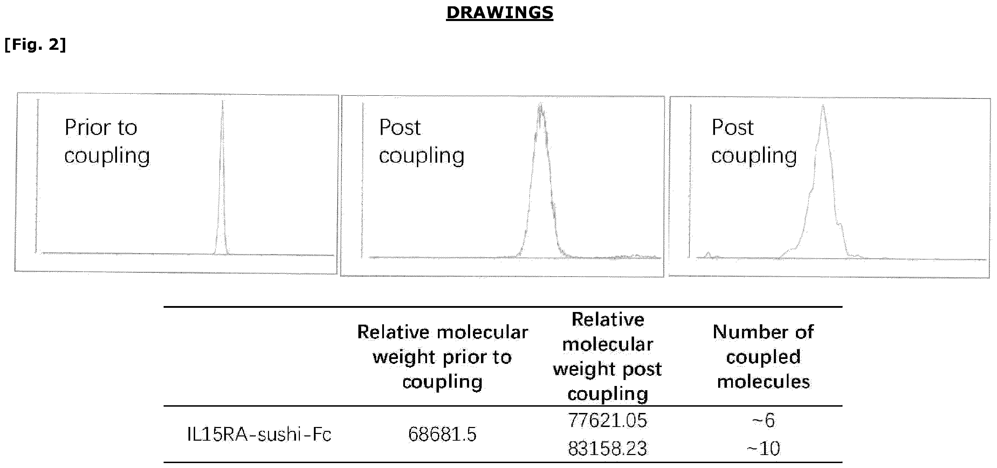

The relative molecular weight of N803 measured by MALDI-TOF before the coupling was 68681.5 Da (Figure 2, left, middle, and right mass spectra were obtained from the relative molecular weight of IL15RA-sushi-Fc in N803 before and after the coupling, respectively). The relative molecular weights of N803 measured after the coupling were 77621.05 Da and 83158.23 Da, respectively. As the GDP fucose derivatives will contribute approximately 1462.5Da of molecular weight after the coupling, the coupled N803 will carry about 6 to 10 GDP fucose derivatives. Unless otherwise specified, N803 means a GDP-fucose coupled N803.

### Example 3. Coupling of human peripheral blood red blood cells with IL-15 derivatives and detection by FACS

Red blood cells were washed with PBS, and serum and other cells were completely removed for later use. 10 µg/mL and 100 µg/mL fucosyltransferase (prepared according to CN114369584A and CN114369585A. In the present disclosure, the recombinant fucosyltransferase mutant of Helicobacter pylori with SEQ ID NO. 13 of CN114369585A was used), 15 µg/mL and 150 µg/mL of N803 were added respectively to 5 × 10⁹/mL of the red blood cells. Incubate at room temperature for 30 minutes, wash with PBS pH 7.4, centrifuge at 500 × g, and remove the supernatant. The red blood cells were resuspended in PBS pH 7.4 and incubated with anti-hIgG Biotin antibody (Cat# F030822, diluted to 1:200, Beijing BioLegend Technology Co., Ltd.) at 4 °C for 30 minutes. After washing once with PBS, they were stained with Streptavidin PE (BioLegend, Inc., Cat# 405204, diluted to 1:200) and incubated at 4 °C for 30 minutes. After washing once with PBS, they were detected using a flow cytometer (MateCyte, Beijing Challen Biotechnology Co., Ltd.) and analyzed using NovoExpress software.

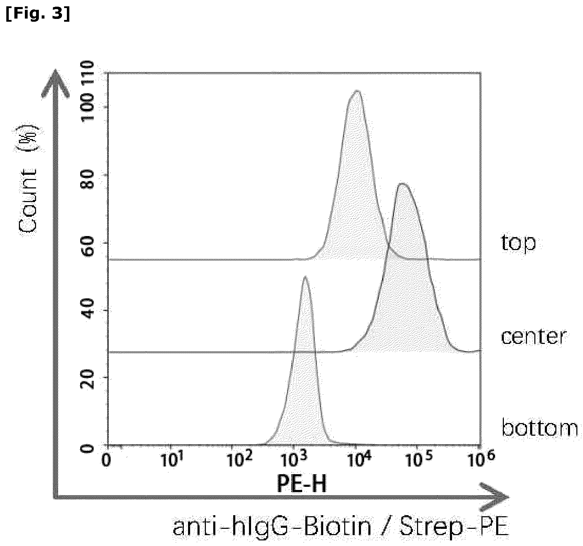

Figure 3 shows a histogram of N803 protein quantity (anti-hIgG-Biotin/Strep-PE) on the red blood cell membrane detected by flow cytometry under the coupling conditions. The red blood cells shown in the top, bottom, and center are: coupled red blood cells under 15 µg/mL N803, 10 µg/mL fucosyltransferase (top), coupled red blood cells under 150 µg/mL N803, 100 µg/mL fucosyltransferase (middle), and uncoupled red blood cells (bottom). Detection of N803: First, use anti-hIgG-Biotin antibody to specifically bind to the human IgG4-Fc sequence of the N803 protein, then use Streptavidin PE (Strep PE) to specifically bind to the Biotin on the anti-hIgG-Biotin antibody, and finally use flow cytometry to detect PE fluorescence. The intensity of PE fluorescence is positively correlated with the quantity of N803. From the figure, it can be seen that PE (N803) could be detected in both red blood cells under the 2 coupling conditions, and there was a clear differentiation from the uncoupled red blood cells. The quantity of the coupled N803 was related to the number of antibodies and enzymes. The greater the number, the greater the amount of coupling.

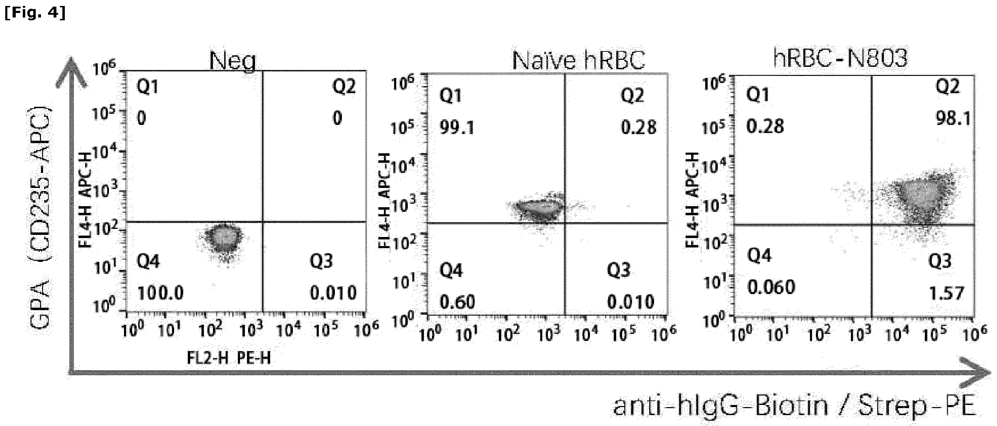

To further determine the cell type, the N803 coupled red blood cells were stained with anti-GPA(CD235)-APC (BioLegend, Inc., Cat# 306608, diluted to 1:1400). GPA is a cell membrane protein to red blood cells. Figure 4 shows the flow cytometry results of uncoupled red blood cells (Naive hRBC, middle), red blood cells coupled with the N803 protein (hRBC-N803, top), and unstained red blood cells serving as a blank control (neg, bottom), the GPA being the y-axis and the N803 (anti-hIgG-PE) being the x-axis. Detection procedure: the uncoupled red blood cells and the red blood cells coupled with the N803 protein were washed once with PBS and stained with anti-GPA(CD235)-APC and anti hIgG-PE antibodies (Jackson ImmunoResearch Inc., Cat#109-116-170, diluted to 1:200) simultaneously. After being co-incubated together at 4 °C for 30 minutes, and washed once with PBS, the red blood cells were detected using a flow cytometer (MateCyte, Beijing Challen Biotechnology Co., Ltd.) and were analyzed using NovoExpress software. As can be seen from Figure 4, compared with the blank control, more than 99% of the red blood cells obtained by the above method were GPA positive , indicating that more than 99% of the cells were red blood cells. Compared with the uncoupled red blood cells, PE (N803) were detected in all coupled red blood cells, and were all coupled with N803.

### Example 4. Mouse red blood cell coupling with IL-15 derivatives and FACS detection to study the influence of red blood cell density, enzyme concentration, drug concentration, temperature, and reaction time on the coupling effect

### 1. Optimization of red blood cell density involved in the reaction conditions of antibody coupled red blood cells

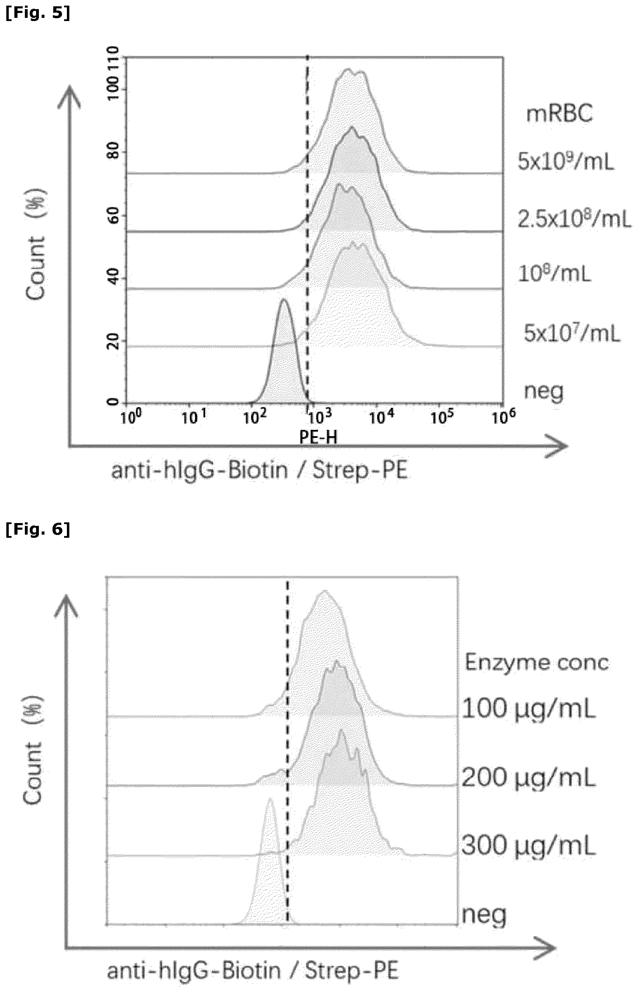

GDP-fucose coupled N803 were prepared according to Example 3. Mouse red blood cells were washed with PBS, and serum and other cells were completely removed for later use. 100 µg/mL was selected as the concentration of fucosyltransferase, and the concentration of N803 was 300 µg/mL. Red blood cells were added to the reaction system at 5 × 10⁹/mL, 2.5 × 10⁸/mL, 10⁸/mL, and 5 × 10⁷/mL, respectively. The reaction system was incubated at room temperature for 30 minutes, followed by washing with PBS pH 7.4, and centrifuging at 500 × g and the supernatant was removed. The red blood cells were resuspended in PBS pH 7.4 and incubated with anti-hIgG-Biotin antibody (Beijing BioLegend Technology Co., Ltd., Cat# F030822, diluted to 1:200) at 4 °C for 30 minutes, washed once with PBS, stained with Streptavidin-PE (BioLegend, Inc., Cat# 405204, diluted to 1:200), and detected by flow cytometry (MateCyte, Beijing Challen Biotechnology Co., Ltd.), followed by analysis using NovoExpress software. The results showed (Figure 5) that the fluorescence brightness was highest when the red blood cell density was 5 × 10⁷/mL, indicating the largest quantity of the coupled N803.

### 2. Optimization of fucosyltransferase concentration in the reaction conditions of the red blood cell coupled with antibody

GDP-fucose coupled N803 was prepared according to Example 3. Mouse red blood cells were washed with PBS, and serum and other cells were completely removed for later use. The red blood cell concentration was 5 × 10⁸/mL, N803 concentration was 600 µg/mL, and fucosyltransferase was added to the reaction system at concentrations of 100 µg/mL, 200 µg/mL, and 300 µg/mL, respectively. The reaction system was incubated at room temperature for 30 minutes, washed with PBS pH 7.4, and centrifuged at 500 × g, and supernatant was removed. The red blood cells were resuspended in PBS pH 7.4 and incubated with anti-hIgG-Biotin antibody (Beijing BioLegend Technology Co., Ltd., Cat# F030822, diluted to 1:200) at 4 °C for 30 minutes, washed once with PBS, stained with Streptavidin PE (BioLegend, Inc., Cat# 405204, diluted 1:200), and then detected by flow cytometry (MateCyte, Beijing Challen Biotechnology Co., Ltd.), followed by analysis using NovoExpress software. The results (Figure 6) showed that when the concentration of fucosyltransferase was between 100 µg/mL and 300 µg/mL, under this reaction condition, N803 could effectively couple to the red blood cells.

### 3. Optimization of drug macromolecule N803 concentration in the reaction conditions of red blood cell coupled with antibody

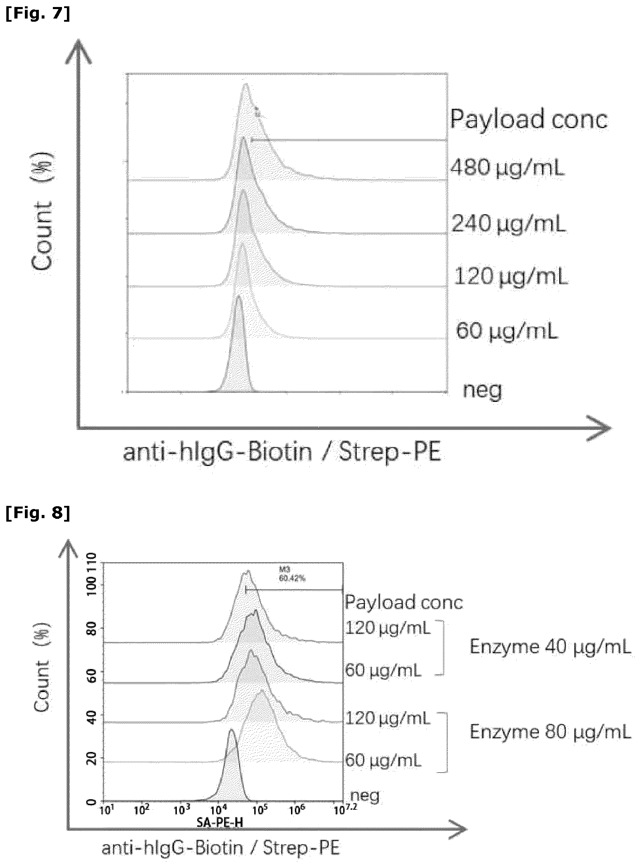

GDP-fucose coupled N803 was prepared according to Example 1. Mouse red blood cells were washed with PBS, and serum and other cells were completely removed for later use. The red blood cell density was 10⁷/mL, and 40 µg/mL or 80 µg/mL was selected as the concentration of fucosyltransferase. N803 was added to the reaction system at concentrations of 60 µg/mL, 120 µg/mL, 150 µg/mL, 240 µg/mL, 300 µg/mL, 480 µg/mL, and 600 µg/mL, respectively. The reaction system was incubated at room temperature for 30 minutes, washed with PBS pH 7.4, centrifuged at 500 × g, and supernatant was removed. The red blood cells were resuspended in PBS pH 7.4 and incubated with anti-hIgG-Biotin antibody (Beijing BioLegend Technology Co., Ltd., Cat# F030822, diluted to 1:200) at 4 °C for 30 minutes, washed once with PBS, stained with Streptavidin PE (BioLegend, Inc., Cat# 405204, diluted to 1:200), and then detected by flow cytometry (MateCyte, Beijing Challen Biotechnology Co., Ltd.), followed by analysis using NovoExpress software. The results (Figures 7-9) showed that when the concentration of N803 was between 60µg/mL- 600µg/mL, under this reaction condition, N803 could effectively couple to the red blood cells.

### 4. Optimization of reaction temperature and reaction time in the reaction conditions of red blood cells coupled with antibodies

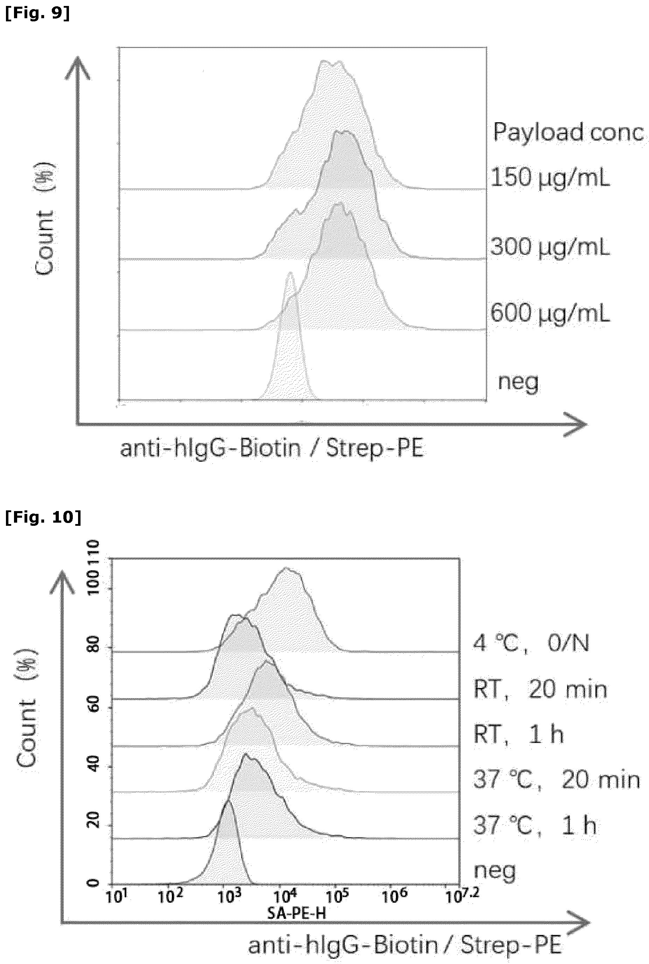

GDP-fucose coupled N803 was prepared according to Example 1. Mouse red blood cells were washed with PBS, and serum and other cells were completely removed for later use. The concentration of the red blood cells was 10⁷/mL, the concentration of fucosyltransferase was 40 µg/mL, and the concentration of N803 was 480 µg/mL. The reaction conditions were 4 °C overnight (approximately 16 hours); 20 minutes or 1 hour at room temperature; 20 minutes or 1 hour at 37 °C, respectively. After the incubation, the reaction system was washed with PBS pH 7.4, and centrifuged at 500 × g, and the supernatant was removed. The red blood cells were resuspended in PBS pH 7.4 and incubated with anti-hIgG-Biotin antibody (Beijing BioLegend Technology Co., Ltd., Cat# F030822, diluted to 1:200) at 4 °C for 30 minutes, washed once with PBS, stained with Streptavidin PE (BioLegend, Inc., Cat# 405204, diluted to 1:200), and then detected by flow cytometry (MateCyte, Beijing Challen Biotechnology Co., Ltd.), followed by analysis using NovoExpress software. The results (Figure 10) show that N803 could be coupled to the red blood cells at all three temperatures. In comparison, a better separation effect could be achieved at room temperature (10 °C to 30 °C), 20 minutes.

### Example 5. Composition of reaction solution and effect of Mg ions on the coupling reaction

According to the literature report [Jie, L., et al. "A Single-Step Chemoenzymatic Reaction for the Construction of Antibody-Cell Conjugates." ACS Central Science 4.12 (2018).], fucosyltransferase is magnesium (Mg) dependent, and 20 mM Mg²⁺ is required in the reaction system when fucosyltransferase catalyzes the glycosyl transfer reaction. However, in the pharmaceutical field, any additives in the drug manufacturing process can affect the quality and safety of the final drug, especially the latter. Changes in the concentration of metal ions in blood may lead to functional disorders of various organs, such as hypermagnesemia, which is manifested at the early stage as loss of appetite, nausea, vomiting, skin flushing, headache, dizziness, etc. Due to the lack of specificity, they are easy to be overlooked. When the serum magnesium concentration reaches 2-4 mM, significant changes in the neuromuscular and circulatory systems can occur. Therefore, removing additives and excipients as much as possible during the pharmaceutical manufacturing process can minimize potential safety hazards of drug use to patients.

Main components of a commercial red blood cell preservation solution are sodium chloride, sodium citrate, citric acid, and glucose. Under 4 °C, the red blood cells can be stored for 2 weeks without changing their activity and characteristics. It is often used for blood collection, preservation, and transportation and does not contain Mg²⁺ (Ye Hanquan et al., Dynamic Observation of Quality Control on Suspension Wash of Red Blood Cells by Red Blood Cell Preservation Solution, Journal of Changjiang University (Natural Science Edition) Medical late monthly Journal, 2013). Experimental studies have shown that adding 20 mM Mg²⁺ to PBS buffer or red blood cell preservation solution can cause rupture and hemolysis of red blood cells even under 4 °C (data not shown).

However, Mg²⁺ is an important cofactor for enzymes, which can stabilize conformation, form the active center of enzymes, and act as a bridge to connect substrate molecules with enzyme proteins. In this field, GDP-Fucose is used as a substrate for fucosyltransferase, in which the phosphate molecule of GDP weakly binds to Mg²⁺, which can reduce the activation energy of the catalytic reaction and promote the reaction to proceed (Simonson, T. and Satpati, P. (2013), Simulating GTP:Mg and GDP:Mg with a simple force field: A structural and thermodynamic analysis. J. Comput. Chem., 34: 836-846.).

In the present study, drugs coupled with GDP Fucose derivatives were used as one of the substrates for fucosyltransferase. The coupled portion is a macromolecule (antibody or fusion protein, abbreviated as GDP-Fucose-antibody, with a molecular weight greater than 50 kD), rather than a natural small molecule substrate (such as the commercialized derivative GDP-Azido-Fucose, GDP azide fucose, with a molecular weight of only 630 Da). Another substrate of fucosyltransferase is polysaccharides on cell membrane glycoproteins such as LacNAc. Under the action of fucosyltransferase, GDP-Fucose-antibody forms glycosidic bonds with LacNAc and forms LacNAc-Fucose-antibody after releasing GDP, thereby coupling the antibody molecule to the sugar chain on the cell membrane. Based on an analysis of the molecular structure, the steric hindrance between fucosyltransferase and GDP-Fucose-antibody is significantly greater than that between fucosyltransferase and a small molecule substrate. The effect of steric hindrance will significantly affect the participation of Mg²⁺, and the function of the Mg²⁺ involved may not be apparent. Considering that free phosphate molecules can act as products to inhibit enzymatic reactions, removing the phosphate portion from conventional buffer solutions can reduce the inhibitory effect of the products and compensate for the negative impact caused by the lack of Mg²⁺. To confirm our hypothesis, we adjusted the conventional PBS buffer (137mM sodium chloride, 10mM phosphate, 2.7mM potassium chloride; pH 7.4) to a phosphate free equilibrium solution and named it NPBS (Non Phosphate Buffer Solution): 150mM sodium chloride, 2.7mM potassium chloride, 44mM glucose. We also adjusted the pH to 5.9-6.3, which is more in line with the *in vitro* survival conditions of red blood cells. Our results showed that Mg²⁺ can be removed in this reaction system without affecting the enzymatic reaction.

Due to the rupture and hemolysis of red blood cells caused by the addition of Mg²⁺, it was not possible to evaluate the enzymatic reaction of the NPBS reaction system with or without Mg²⁺. Therefore, we used the human embryonic kidney cell line (HEK293) for system verification.

After being washed with PBS, the human embryonic kidney cell line (HEK293) was completely separated from the culture medium for later use. The conditions for coupling 3H3 with HEK293 cells were the same as those for coupling N803 with red blood cells. The concentration of fucosyltransferase was 100 µg/mL, the concentration of 3H3 was 120 µg/mL, the cell density of HEK293 was 5 × 10⁷/mL, and NPBS (without Mg²⁺) and NPBS (with 20 mM Mg²⁺ added) were selected as the reaction buffer, respectively. After incubation at room temperature for 30 minutes, followed by centrifuging at 200×g and removal of the supernatant, the HEK293 cells coupled with 3H3 (HEK293-3H3) were resuspended in PBS, and incubated with anti-hIgG-Biotin antibody (Beijing BioLegend Technology Co., Ltd., Cat# F030822, diluted to 1:200) at 4 °C for 30 minutes, washed once with PBS, stained with Streptavidin PE (BioLegend, Inc., Cat# 405204, diluted to 1:200), and then detected by flow cytometry (NovoCyte, Agilent Technologies (China) Co., Ltd), followed by analysis using NovoExpress software.

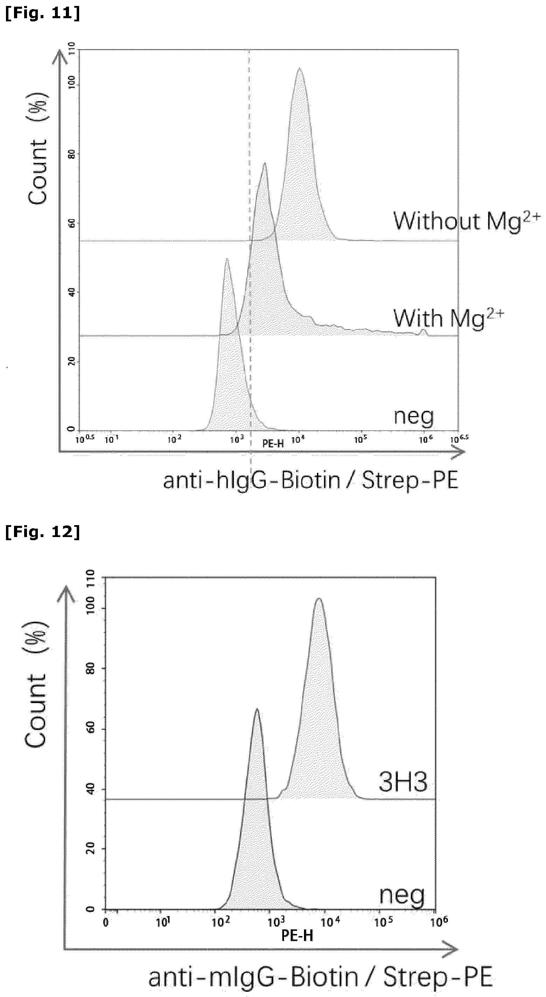

Figure 11 shows a histogram of the amount of 3H3 protein (anti-mIgG-Biotin/Strep-PE) on the membrane of HEK293 cells detected by flow cytometry under the coupling conditions. The HEK293 cells shown in the top, middle, and bottom are respectively: HEK293 cells coupled in NPBS (without Mg²⁺) reaction buffer (top), HEK293 cells coupled in NPBS (with 20 mM Mg²⁺added) reaction buffer (middle), and uncoupled HEK293 cells (bottom, neg). Detection of 3H3: first, anti-mIgG-Biotin antibody was used to specifically bind to the mouse IgG2aa sequence of 3H3 protein. Then Streptavidin-PE (abbreviated as Strep-PE) was used to specifically bind to Biotin on the anti-mIgG-Biotin antibody. Finally, flow cytometry was used to detect PE fluorescence, and the intensity of PE fluorescence was positively correlated with the amount of 3H3. From the figure, it can be seen that PE (3H3) could be detected in both red blood cells coupled under two different coupling conditions, which was clearly distinguishable from the uncoupled red blood cells. Moreover, the fluorescence brightness of 3H3 on the HEK293 cells coupled under Mg²⁺ free buffer was increased by about 20% compared to the fluorescence brightness of 3H3 on the HEK293 cells coupled under 20 mM Mg²⁺ buffer, indicating that more antibody molecules were coupled to the cell membrane, that is, the coupling efficiency using NPBS (without Mg²⁺) buffer was better.

As a reaction solution composition for fucosyltransferase, unless otherwise specified, NPBS buffer without Mg²⁺was used in the red blood cell coupling reaction system. Other reaction conditions (such as flow cytometry detection, ATP concentration detection, 2,3-DPG concentration detection, as well as cleaning, storage, functional detection of red blood cell coupling products, and detection of various non red blood cells) were performed using PBS buffer.

### Example 6. Coupling of Mouse Red Blood Cell with Anti-Mouse 4-1BB Antibody and Detection by FACS

33H3 is an anti-mouse 4-1BB antibody [Rickert, K. W. et al. Combining phage display with de novo protein sequencing for reverse engineering of monoclonal antibodies. MAbs 8501-512 (2016)]. Based on the sequences published in the literature, the corresponding DNA was designed and fused with the constant region sequence of mouse IgG2aa. Suzhou Biotechnology Co., Ltd. was commissioned to synthesize plasmid DNA and clone it into vector pRM293 (pRM293 was obtained by modifying plasmid pTT5, see Shi C. Purification and characterization of a recombinant G-protein coupled receptor, Saccharomyces cerevisiae Ste2p, transiently expressed in HEK293 EBNA1 cells. Biochemistry. 2005; 44 (48): 15705-15714.). Also Suzhou Biotechnology Co., Ltd. was commissioned to transiently transfect the plasmid into HEK293 cells (National Research Council, Canada) and perform affinity purification using Mabselect sure (Protein A, GE healthcare). The GDP-fucose derivative (GDP-Fucose-Triazole-PEG4-Tz, unless otherwise specified, all GDP-fucose derivatives used in this study were GDP-Fucose-Triazole-PEG4-Tz) was coupled with purified 3H3 via an amine reaction (the coupling method was the same as the N803 coupling method mentioned above) for later use. Unless otherwise specified, all 3H3 was 3H3 coupled with GDP-fucose.

100 µg/mL was selected as the concentration of fucosyltransferase, the concentration of 3H3 was 200 µg/mL, and the red blood cell density added to the reaction system was at 2 × 10⁹/mL. After being incubated at room temperature for 30 minutes, the red blood cells labeled with 3H3 (mRBC-3H3) were obtained. The supernatant was removed by centrifugation at 500 × g. mRBC-3H3 was resuspended in PBS pH 7.4 for subsequent FACS detection, incubated with anti-mouse IgG-Biotin antibody (Beijing BioLegend Technology Co., Ltd., Cat# F030805, diluted to 1:200) at 4 °C for 30 minutes, washed once with PBS, stained with Streptavidin-PE (BioLegend, Inc., Cat# 405204, diluted to 1:200), and then detected by flow cytometry (MateCyte, Beijing Challen Biotechnology Co., Ltd.). NovoExpress software was used for the analysis. The results (Figure 12) showed that 3H3 could effectively be coupled to the red blood cells under this reaction condition. Figure 12 shows a histogram of the amount of 3H3 protein (anti-mIgG-Biotin/Strep-PE) on the membrane of red blood cells detected by flow cytometry under the coupling conditions. The red blood cells shown above and below are: 3H3-coupled red blood cells (above), uncoupled red blood cells (below, neg). Detection of 3H3: first, anti-mIgG-Biotin antibody was used to specifically bind to the mouse IgG2aa sequence of 3H3 protein. Then Streptavidin-PE (Strep-PE) was used to specifically bind to biotin on the anti-mIgG-Biotin antibody. Lastly, flow cytometry was used to detect PE fluorescence, and the intensity of PE fluorescence was positively correlated with the amount of 3H3. From the figure, it can be seen that when compared with the uncoupled red blood cells, PE (3H3) could be detected in the coupled red blood cells with good differentiation from the uncoupled red blood cells, that is, 3H3 could be effectively coupled to red blood cells under this reaction condition.

### Example 7. Coupling of Mouse Red Blood Cell with Anti-human VEGF-Antibody (avastin) and Detection by FACS

Avastin (Bevacizumab), a humanized monoclonal antibody IgG1 developed by Roche, is a vascular endothelial growth factor (VEGF) inhibitor, and was purchased from Targeted Mol Chemicals Inc. [2020-Bevacizumab (Avastin^{®}) in cancer treatment A review of 15 years of clinical] . The GDP-fucose was coupled with purified Avastin via an amine reaction (coupling method was the same as the N803 coupling method mentioned above) for later use. Unless otherwise specified, all Avastin was Avastin coupled with GDP-fucose. 100µg/mL was selected as the concentration of fucosyltransferase, the concentration of Avastin was 200 µg/mL and 1.5 mg/mL, and the red blood cell density added to the reaction system was at 10⁸/mL. After incubation at room temperature for 30 minutes, the red blood cells labeled with Avastin (mRBC-Avastin) were obtained. The supernatant was removed by centrifugation at 500 × g. mRBC-Avastin was resuspended in PBS pH 7.4 for subsequent FACS detection, incubated with anti-mouse IgG- Biotin antibody (Beijing BioLegend Technology Co., Ltd., Cat# F030822, diluted to 1:200) at 4 °C for 30 minutes, washed once with PBS, stained with Streptavidin-PE (BioLegend, Inc., Cat# 405204, diluted to 1:200), and then detected by flow cytometry (MateCyte, Beijing Challen Biotechnology Co., Ltd.). NovoExpress software was used for the analysis.

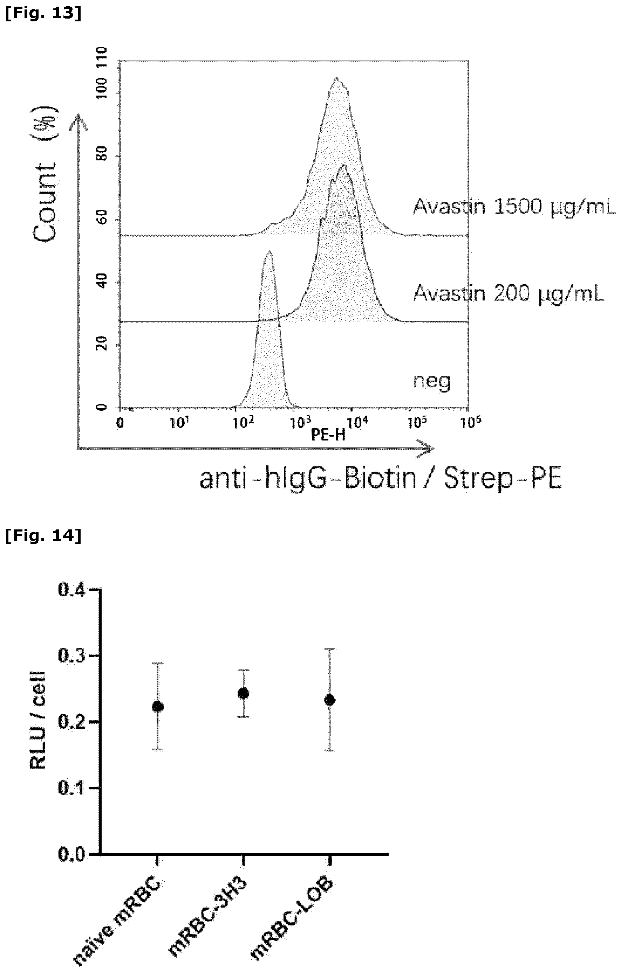

Figure 13 shows a histogram of the amount of Avastin protein (anti-hIgG-Biotin/Strep PE) on the membrane of red blood cells detected by flow cytometry under the coupling conditions. The red blood cells shown on top, middle and bottom are respectively: coupled under condition of 1500 µg/mL Avastin (top), coupled under condition of 200 µg/mL Avastin (top), uncoupled red blood cells (bottom, neg). Detection of Avastin: first, anti-hIgG-Biotin antibody was used to specifically bind to Avastin protein (human IgG1). Then Streptavidin-PE (Strep-PE) was used to specifically bind to biotin on the anti-hIgG-Biotin antibody. Finally, flow cytometry was used to detect PE fluorescence, and the intensity of PE fluorescence was positively correlated with the amount of Avastin. From the figure, it can be seen that compared with the uncoupled red blood cells, PE (Avastin) could be detected in the coupled red blood cells under the 2 coupling conditions. There was not much difference in the intensity of PE fluorescence for the red blood cells coupled under 200 µg/mL Avastin and under 1500 µg/mL Avastin, that is, Avastin could be effectively coupled to red blood cells under the two reaction conditions.

### Example 8. Evaluation of the self-function of red blood cells when engineered red blood cells are used as therapeutic drugs

This example demonstrates that the described method of the present disclosure does not have a destructive effect on red blood cells and does not affect the function of red blood cells themselves.

The lifespan of red blood cells is 100-130 days, with an average of around 125 days; the lifespan of red blood cells in mice is around 30-45 days. Normal red blood cells have no nuclei, no organelles, are in the shape of a double concave circular disc, are plastic and deformable, and have the ability to carry oxygen. Blood transfusion is the earliest cell therapy used in clinical practice, mainly for treating anemia. Maintaining the normal function of red blood cells outside the body is a hot topic in transfusion research, with the aim of enabling transfusion-grade red blood cells to maintain their normal lifespan and oxygen-carrying function *in vivo.* The most commonly used methods for evaluating red blood cell function include measuring the content of ATP, 2,3-DPG, free hemoglobin in the red blood cells , and the red blood cell deformability.

### 1. ATP content in red blood cells

Mature red blood cells do not have any organelles such as mitochondria, and cannot produce ATP through mitochondria for energy supply like most cells. Red blood cells produce ATP mainly through glucose fermentation, i.e. anaerobic respiration, which is used to maintain ion pumps (sodium pump, calcium pump) on the red blood cell membrane, to maintain ion balance of red blood cells and maintain membrane plasticity and its special double concave disc shape. If lacking ATP, the ion balance between inside and outside of the red blood cell membrane will be lost, and then more Na⁺ will enter into the cell more than the discharge of K⁺, and more Ca²⁺ will also enter. The red blood cells absorb too much water and swell into spherical shapes, and even rupture . Therefore, the ATP level inside the red blood cells is crucial for maintaining their normal morphology, deformability, and oxygen carrying function.

ATP detection kit (Cat# S0026, Beyotime Biotechnology Co., Ltd.) was developed based on the theory that when firefly luciferase catalyzes luciferin to produce fluorescence, it needs ATP to provide energy. When both firefly luciferase and luciferin are excessive, the production of fluorescence within a certain concentration range is proportional to the concentration of ATP. The experimental procedure is described as follows: 10⁶ red blood cells are centrifuged and precipitated, the supernatant is discarded, the cells are gently dispersed, and 20 µL of red blood cell lysis solution was added. Vortex properly to fully lyse the cells. After the lysis, centrifuge at 12,000g at 4 °C for 5 minutes, take the supernatant, and place it on ice for subsequent measurements. The ATP detection reagent contains luciferase and substrate. After appropriate dilution, the sample obtained from the red blood cell lysis is added and quickly mixed using a micropipette. After being left for at least 2 seconds, RLU is measured using a chemiluminescence analyzer (cartridge SpectraMax Paradigm multifunctional enzyme-linked immunosorbent assay reader, Molecular Devices Instrument (Shanghai) Co., Ltd.), and the RLU value of individual cells is calculated (Figure 14). Figure 14 shows that mouse red blood cells were coupled with anti-mouse 4-1BB antibody 3H3 and anti-mouse 4-1BB antibody LOB respectively. After washing and validation by flow cytometry, the ATP content in the red blood cells was detected according to the above procedure. The results showed that compared with natural red blood cells without the coupled antibodies, there was no significant change in the ATP content in the red blood cells coupled with the antibodies. 3H3 and LOB 12.3 (hereafter abbreviated as LOB) are anti-mouse 4-1BB antibodies [Rickert, K. W. et al. Combining phage display with de novo protein sequencing for reverse engineering of monoclonal antibodies. MAbs 8,501-512 (2016).] .

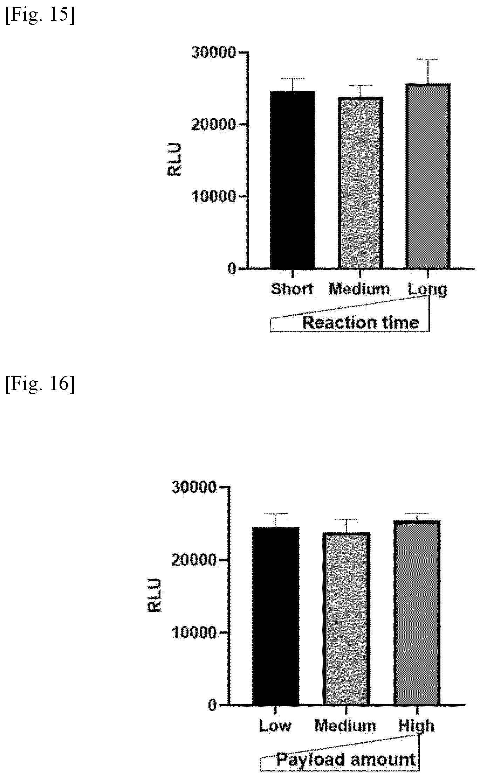

In addition, under the conditions of cell density of 5 × 10⁹/mL, fucosyltransferase concentration of 100 µg/mL, antibody or Fc fusion protein concentration of 200 µg/mL, and incubation time of 30 minutes, 60 minutes, and 120 minutes, the ATP content in the red blood cells coupled with the antibodies was determined. As shown in Figure 15, a prolonged incubation time had no significant effect on the ATP content in the red blood cells.

Under the conditions of cell density of 5 × 10⁹/mL, fucosyltransferase concentration of 100 µg/mL, incubation time of 30 minutes, and antibody or Fc fusion protein concentrations of 100µ g/mL, 200 µg/mL, and 400 µg/mL, the ATP content in the red blood cells coupled with antibodies was determined. As shown in Figure 16, the concentration of antibody or Fc fusion protein had no significant effect on the ATP content in the red blood cells between 100 µg/mL and 400 µg/mL.

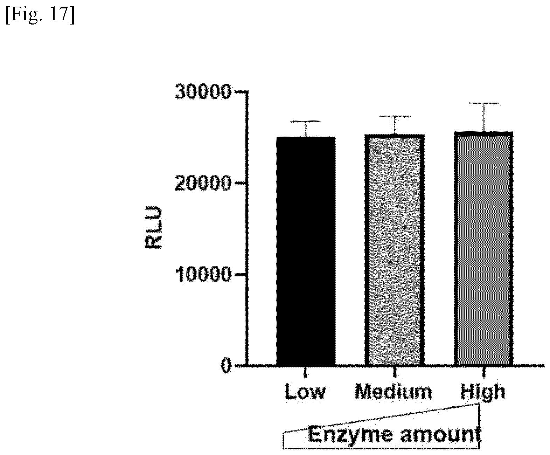

Under the conditions of cell density of 5 × 10⁹/mL, antibody or Fc fusion protein concentration of 200 µg/mL, incubation time of 30 minutes, and fucosyltransferase concentrations of 50 µg/mL, 100 µg/mL, and 200 µg/mL, the ATP content in the red blood cells coupled with the antibodies was determined. As shown in Figure 17, the concentration of fucosyltransferase had no significant effect on the ATP content in the red blood cells between 50 µg/mL and 200 µg/mL.

### 2. 2,3-DPG content in red blood cells

2,3-diphosphoglycerate (2,3-DPG) in red blood cells is an intermediate product of glycolysis metabolism and is often used as a marker of the oxygen carrying capacity of red blood cells. When it binds to hemoglobin (Hb), the affinity of Hb for oxygen is reduced, thereby promoting the release of oxygen from oxygenated Hb. There are reports indicating that when blood is stored at 4 °C for about a week, 2,3-DPG will basically disappear, and its decrease will shift the oxygen dissociation curve to the left, affecting the tissue's utilization of oxygen. Therefore, the level of 2,3-DPG reflects the oxygen carrying capacity of red blood cells and determines the ease of hemoglobin releasing oxygen in tissues.

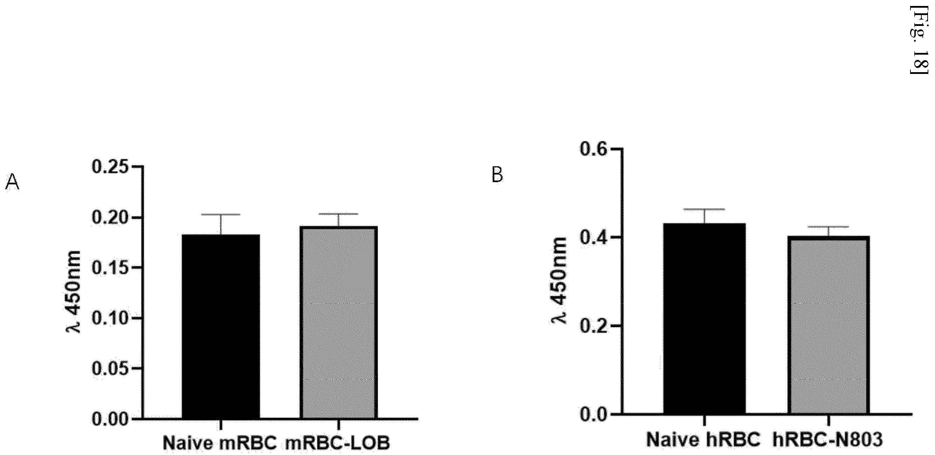

Enzyme-linked immunosorbent assay kit for mouse 2,3-DPG (Jiangsu Jingmei Biotechnology Co., Ltd.) was used to determine the content of 2,3-DPG in the sample by double antibody sandwich method. A microwell is coated with purified mouse 2,3-DPG antibody to prepare a solid-phase antibody. Add the test sample and HRP labeled 2,3-DPG antibody sequentially into the microwell to form an antibody-antigen-enzyme-linked antibody complex. The final chromogenic substrate exhibits varying shades of color under the catalysis of HRP, which is positively correlated with the content of 2,3-DPG. The operation procedure is described as follows: 10⁷ red blood cells are centrifuged and precipitated, the supernatant is discarded, and 50 µL of red blood cell lysis solution is added. After the lysis, centrifuge at 12,000g at 4 °C for 5 minutes. A supernatant is obtained and added into the coated microwell, and incubate at 37 °C for 30 minutes. After a thorough washing, add the enzyme labeled antibody and incubate at 37 °C for 30 minutes. After a thorough washing, add a color developing solution. After 10 minutes of color development at 37 °C, add a stop solution. Absorbance (OD value) is read at a wavelength of 450 nm within 15 minutes (Figure 18). Figure 18-A shows that there was no significant change in the content of 2,3-DPG in the antibody coupled red blood cells compared to natural red blood cells not coupled with the antibody.

Enzyme-linked immunosorbent assay kit for human 2,3-DPG was purchased from Wenzhou Kemiao Biotechnology Co., Ltd. The principle and results are similar to the mouse red blood cell test. Figure 18-B shows that compared to natural red blood cells not coupled with the antibody, there was no significant change in the content of 2,3-DPG in the protein coupled red blood cells.

### 3. Free hemoglobin content

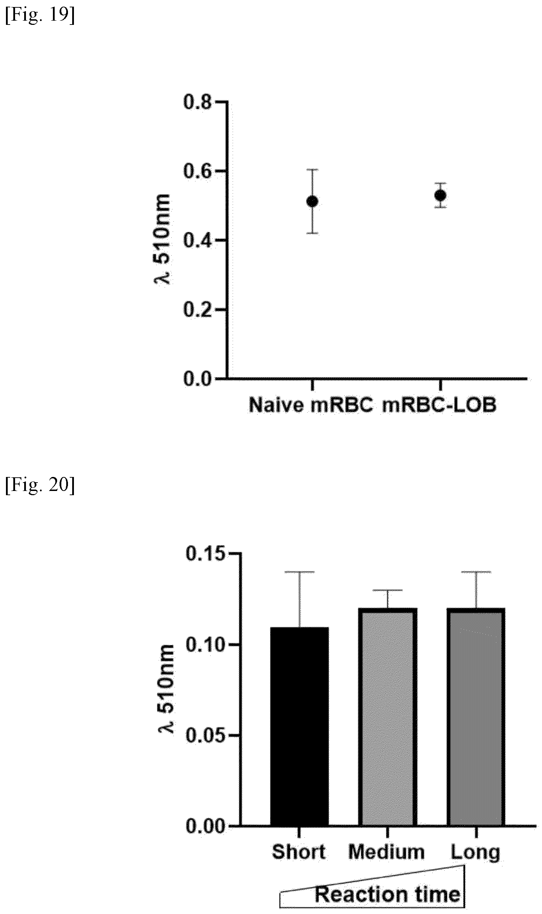

Hemoglobin (Hb) exists in red blood cells. When red blood cells are damaged, hemoglobin is released into the bloodstream, and then there is an increase in free hemoglobin in the plasma. Therefore, the level of free hemoglobin reflects the integrity of red blood cells. The principle of the trace free hemoglobin assay kit (Beijing BioLegend Technology Co., Ltd.) is based on the peroxidase-like activity of ferroheme in hemoglobin molecules, which catalyzes the release of new ecological oxygen from H₂O₂, causing phenol and 4-AAP to oxidize into red substances. The color depth is proportional to the Hb content. The operation process is described as follows: a color reagent is prepared and used according to the ratio provided in the instructions, and mix at the ratio of color reagent: sample = 50:3. The absorbance (OD value) at 510 nm wavelength is read after 20 minutes of water bath at 37 °C. As shown in Figure 19, 5 × 10⁶ mouse red blood cells coupled with anti-mouse 4-1BB antibody LOB were resuspended in 50 µL PBS solution, centrifuged at 12,000g for 5 minutes, and the supernatant was taken. The free hemoglobin content in the supernatant was detected according to the above procedure. The results showed that the red blood cells coupled with the antibody did not undergo significant hemolysis or there was no apparent release of free hemoglobin compared to natural red blood cells.

In addition, under the conditions of cell density of 5 × 10⁹/mL, fucosyltransferase concentration of 100 µg/mL, antibody or Fc fusion protein concentration of 200 µg/mL, and incubation time of 30 minutes, 60 minutes, and 120 minutes, the concentration of free hemoglobin was detected, respectively. As shown in Figure 20, a prolonged incubation time had no significant effect on the integrity of the red blood cell membrane.

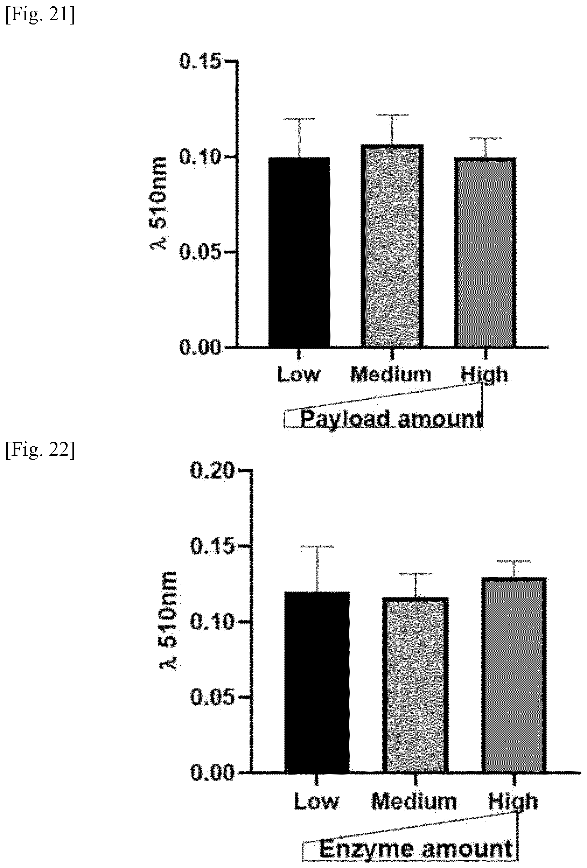

Under the conditions of cell density of 5 × 10⁹/mL, fucosyltransferase concentration of 100 µg/mL, incubation time of 30 minutes, and antibody or Fc fusion protein concentrations of 100 µg/mL, 200 µg/mL, and 400 µg/mL, the concentration of free hemoglobin was detected separately. As shown in Figure 21, the concentration of the antibody or Fc fusion protein had no significant effect on the integrity of red blood cell membranes between 100 µg/mL and 400 µg/mL.

Under the conditions of cell density of 5 × 10⁹/mL, antibody or Fc fusion protein concentration of 200 µg/mL, incubation time of 30 minutes, and fucosyltransferase concentrations of 50 µg/mL, 100 g/mL, and 200 µg/mL, the concentration of free hemoglobin was detected. As shown in Figure 22, the concentration of fucosyltransferase had no significant effect on the integrity of the red blood cell membrane between 50 µg/mL and 200 µg/mL.

### 4. Deformability of red blood cell

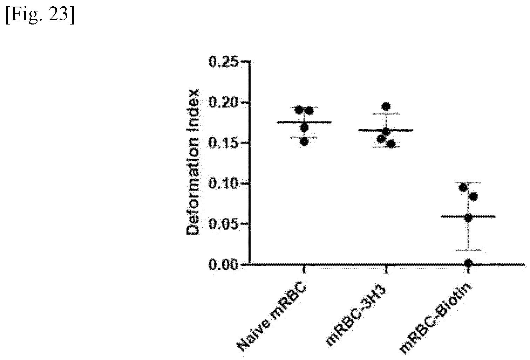

Deformability of red blood cell is one of the important factors affecting the apparent viscosity of blood and effective perfusion of microcirculation in the body, and it is also an important determinant of red blood cell lifespan. At present, there are many methods for measuring the deformability of red blood cells, which can be basically divided into two categories: the first category is to use red blood cell suspensions to compare the average deformability of red blood cell populations, such as laser diffraction method; the second category is to use a single red blood cell to determine its deformability and mechanical characteristics of the cell membrane, such as bottom adhesion method, micropipette method, electron spin resonance spectroscopy, etc. The present disclosure utilizes laser diffraction method (LBY-BX red blood cell deformameter, Beijing Precil Instrument Co., Ltd.) to evaluate the average deformability of red blood cell populations. At different shear rates, deformation index (DI), which measures the percentage of red blood cells elongated in a certain suspension medium using a laser diffractometer, can reflect the deformability of red blood cells. As shown in Figure 23, two different methods for labeling red blood cells were compared. The experimental process is briefly described as follows.

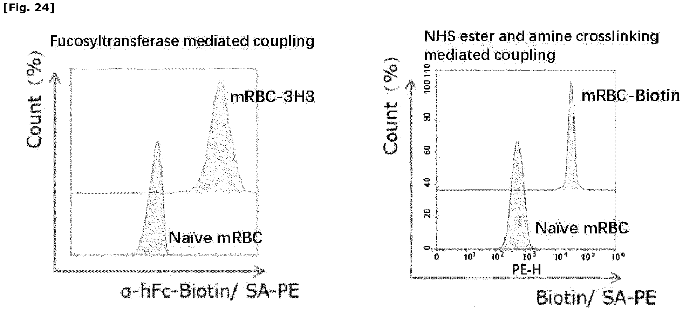

The conditions of coupling mouse red blood cell mediated by fucosyltransferase in the present disclosure are cell density of 5 × 10⁹/mL, antibody 3H3 protein concentration of 200 µg/mL, fucosyltransferase concentration of 100 µg/mL, and incubation time of 30 minutes at room temperature. After being washed, the red blood cells are named as mRBC-3H3. To contrast, we purchased Sulfo-NHS-Biotin (Beijing BioLegend Technology Co., Ltd. Cat# GS4320) and labeled Biotin onto the lysine in the protein molecules on the surface of the red blood cell membranes using the classic method of crosslinking N-hydroxysuccinimide ester (NHS ester) with amines. This is also a common method for labeling most cell membranes, such as the method described in Rubius Therapeutics' 2021 article for labeling red blood cells (2021-Anti-tumor effects of RTX-240 an engineered red blood cell); and in 1987, labeling mouse red blood cells with biotin was used to measure the lifespan of red blood cells in mice (1987-Biotinylated Erythrocytes In Vivo Survival and In Vitro Recovery). We used the same cell density, 30 µg/mL of Sulfo NHS Biotin, incubated at room temperature for 30 minutes and washed. The red blood cells coupled by this method are named as mRBC-Biotin. The efficiency and homogeneity of labeling of red blood cells obtained by the two coupling methods were detected by flow cytometry (Figure 24), and both methods were able to obtain engineered red blood cells with ideal homogeneity. Non-drug coupled mouse red blood cells were also incubated in PBC solution at the density of 5 × 10⁹/mL at room temperature for 30 minutes and washed, but without the addition of the antibody, fucosyltransferase, and Sulfo NHS Biotin. These cells were named as Naive mRBC.

Under the same conditions, the percentage of red blood cells elongated in PBS suspension medium was measured using a laser diffractometer. As shown in Figure 23, the deformation index of coupled mouse red blood cells (Naive mRBC) ranged from 0.15 to 0.2, which was used as a relatively normal deformation index in this experiment. The coupled mouse red blood cell (mRBC-3H3) mediated by fucosyltransferase were similar to the normal red blood cells and also ranged from 0.15 to 0.2. However, the deformation index of the red blood cells (mRBC-Biotin) treated with the classical amine crosslinking method was around 0.05, and the proportion of deformable cells was significantly reduced. Therefore, compared with natural red blood cells not coupled with the antibody, the deformability of antibody-coupled red blood cells mediated by fucosyltransferase in the present disclosure was not significantly affected.

### Example 9. Evaluation on half-life of mouse red blood cells coupled with antibodies in mice

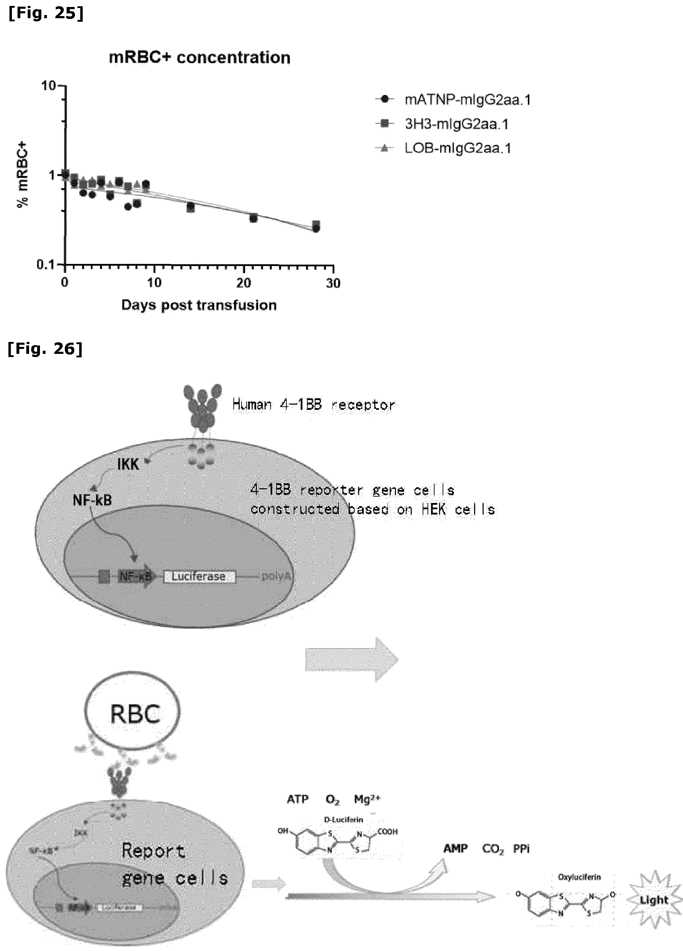

The lifespan of human peripheral blood red blood cells is about 120 days, while the lifespan of mouse peripheral blood red blood cells is around 20-40 days (1958-The Life Span of Red Cells in the Rat and the Mouse as Determined by Labeling with DFP in vivo; 2015-Determination of RBC Survival in C57BL6 and C57BL6-Tg(UBC-GFP) mice). Long half-life is one of the advantages of red blood cells as drug carriers. The strategy of the red blood cell coupling with a drug provided by the present disclosure, as shown in the series of studies in Example 8, does not cause significant damage to the self-function of red blood cells. This characteristic is also reflected in the *in vivo* mice experiments, where the half-life of red blood cells coupled with a drug is not significantly shortened. Mouse red blood cells were coupled with anti-mouse 4-1BB antibody 3H3 and LOB, as well as control antibody mATNP (anti-trinitrophenyl antibody), according to the methods described in Example 3 and Example 4, respectively (P Barber, M.B Rittenberg, Anti-trinitrophenyl (TNP) antibody detection by neutralization of TNP-coliphage T4, Immunochemistry, Volume 6, Issue 2, 1969, Pages 163-174). 5 × 10⁸ were intravenously transfused to the mice, and from the first day after transfusion, blood was drawn from the inner canthus vein of the mice. Flow cytometry analysis was performed according to the staining methods described in Examples 2 and 3. The results showed that until the end of 28 days of the experiment after transfusion, red blood cells coupled with the antibodies could still be clearly detected in peripheral blood (Figure 25). Calculated by nonlinear regression, the half-life of red blood cells coupled with the antibodies was more than 20 days, which is close to the lifespan of normal mouse red blood cells. This indicates that the coupling method described in the present disclosure has no effect on the lifespan of mouse red blood cells. Rubius Therapeutics coupled protein molecules onto the surface of cell membranes by the method of NHS ester cross-linking with amine, and transfused 1 × 10⁸ and 1 × 10⁹ red blood cells twice a week into mice to test half-life and pharmacokinetics in 14 days (Anti-tumor effects of RTX-240 an engineered red blood cell, Cancer Immunol Immunother 2021 Sep;70(9):2701-2719). Compared with Rubius Therapeutics, the number of red blood cells transfused to the mice in this disclosure was similar to the 1 × 10⁸ group of Rubius Therapeutics, only 1/8 of the 1 × 10⁹ group of Rubius Therapeutics. However, the results showed that after 14 days in the 1 × 10⁸ group of Rubius Therapeutics, only about 1% of the red blood cells coupled with drug remained, and only about 15% remained in the 1 × 10⁹ group. Although there is no specific data disclosed, the half-life was significantly shorter than 14 days. The half-life of the antibody-coupled red blood cells mediated by fucosyltransferase in the present disclosure was not significantly affected. The half-life of mATP-mIgG2aa1.1 (abbreviated as mATNP), LOB-mIgG2aa.1 (abbreviated as LOB), and 3H3-mIgG2aa.1 (abbreviated as 3H3) were all close to 30 days, as analyzed using nonlinear two-phase decay in Graphpad prism software, similar to the lifespan of normal mouse red blood cells in peripheral blood . MIgG2aa1.1 is a mutant on the amino acid of the Fc portion of mouse IgG2aa, which reduces the function of Fc binding complement and FcgR (Kovarik J. Highly reduced binding to high and low affinity mouse Fc gamma receptors by L234A/L235A and N297A Fc mutations engineered into mouse IgG2a. Mol Immunol. 2015 Feb;63(2):456-63.).

### Example 10. Functional evaluation on mouse red blood cells as immunomodulatory drugs when coupled with anti-4-1BB antibody

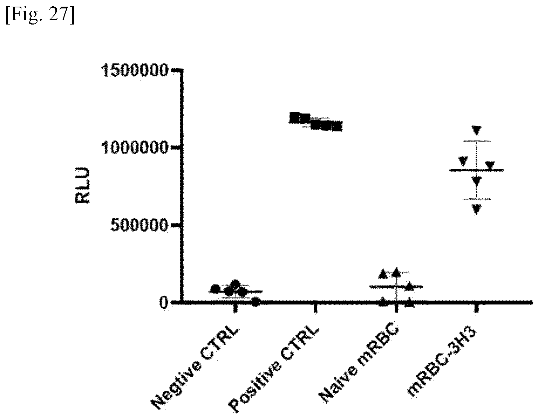

3H3 is an activating antibody against mouse 4-1BB, also known as CD137. 4-1BB, which belongs to the tumor necrosis factor (TNF) receptor family and is expressed on the surface of immune cell membranes such as CD4⁺and CD8⁺ T cells, NKT, NK cells, DCs, macrophage cell etc. When 4-1BB binds to a ligand or an activating antibody, it phosphorylates the I κ B/p65/p50 trimer in the cytoplasm, releasing p65/p50 (NF - κ B), which rapidly enters the nucleus from the cytoplasm and binds to specific sequences on the nuclear DNA, promoting transcription of related genes and facilitating the production and secretion of cytokine. We constructed a reporter gene system for *in vitro* detection of 4-1BB activation by utilizing the characteristics of the signal transduction pathway of 4-1BB. In short, mouse 4-1BB protein was expressed on the cell membrane surface of the human embryonic kidney cell line (HEK293), and a DNA sequence specifically recognized by NF - κ B was introduced into the cell as a promoter. The downstream gene was the luciferase reporter gene (Luc), as shown in Figure 26. When 4-1BB on the cell membrane receives an activating signal, luciferase reporter genes in downstream are expressed, and the expression level of luciferase is proportional to the intensity of transcription factor action. When specific luciferase substrates are added, luciferase reacts with the substrates to produce fluorescence. By detecting the intensity of fluorescence, the activity of luciferase can be determined, thereby determining whether 4-1BB is activated. The luciferase reporter gene pGL4.32 (CAT #. E8491) was purchased from Promega Corporation, an affiliate of Promega (Beijing) Biotech Co., Ltd. The mouse 4-1BB expression plasmid was commissioned to Suzhou Biotechnology Co., Ltd for synthesis. HEK293 cells carrying the reporter gene were transiently transfected with pGL4.32 plasmid and plasmid expressing mouse 4-1BB. After 24 hours, natural mouse red blood cells and mouse red blood cells coupled with 3H3 antibody were added, and incubated for 6 hours. The fluorescence was calculated, where a cassette SpectraMax Paradigm multifunctional enzyme-linked immunosorbent assay reader (Meigu Molecular Instrument (Shanghai) Co., Ltd) was used, and the Bio GloTM luciferase assay system (Promega Corporation, CAT #. G7940) was used for quantitative luminescence (Relative Light Units, RLU). Figure 27 shows that mouse red blood conjugated with 3H3 antibody could effectively activate mouse 4-1BB. 1 × 10⁵ HEK293 cells transfected with mouse 4-1BB plasmid DNA were added 1 × 10⁶ natural mouse red blood cells and mouse red blood cells coupled with 3H3 antibody, respectively, labeled as Naive mRBC and mRBC-3H3; no mouse red blood cells were added as a negative control, marked as Negative CTRL; 1 × 10⁵ HEK293 cells were transfected with pGL4.32 plasmid DNA containing CMV promoter. Without any external stimulation, the cells also showed the expression of luciferase reporter genes downstream. As a positive control, it was labeled as Positive CTRL. The results showed that the RLUs of the negative control, positive control, Naive mRBC, and mRBC-3H3 groups were 7.1 × 10⁴ ± 4.1 × 10^{4,} 1.2×10⁶±2.7×10⁴, 1.0×10⁵±9.3×10⁴, 8.6× 10⁵±1.9×10⁵ respectively. Compared to Naive RBC, mouse red blood cells coupled with 3H3 antibody showed an 8.6-fold increase in activation of the reporter gene. In an *in vitro* detection model, mouse red blood cells coupled with 3H3 antibodies were able to activate the 4-1BB ligand in the mice, suggesting the possibility of these red blood cells serving as immunomodulatory drugs. In the subsequent Example 11, this possibility was further confirmed using mouse primary lymphocytes, and in the *in vivo* experiment of Example 12, anti-tumor properties were also demonstrated.

### Example 11. Evaluation of the function of mouse red blood cells coupled with anti-mouse 4-1BB antibody as an immunomodulatory drug stimulating CD8⁺cells in vitro

Cytotoxic T lymphocytes (CTLs), commonly known as CD8⁺ T cells, are a key component of the adaptive immune system and play an important role in immune defense against intracellular pathogens such as viruses, bacteria, and tumors. In order to confirm that mouse red blood cells coupled with anti-mouse 4-1BB antibodies can serve as immunomodulatory drugs and activate the immune system, especially CD8⁺ T cells and NK cells involved in tumor killing, we isolated and prepared mouse spleen lymphocytes, and detected the response of mouse spleen lymphocytes *in vitro* to mouse red blood cells coupled with anti-mouse 4-1BB antibodies. The process is described as follows: euthanize the mouse by removing the vertebrae, disinfect the lower abdomen of the mouse with alcohol cotton balls, perform aseptic laparotomy, remove the spleen from the abdomen, place it in a culture dish containing PBS buffer, wash and remove excess tissue; cut the spleen into three sections, place it on a 200 mesh nylon sieve by sterile forceps, and place it in a small dish containing 5 mL of fresh PBS buffer. Use a 5 mL syringe plunger to rotate and grind the mouse spleen; remove the nylon sieve and collect the cell suspension into a 15 mL centrifuge tube. Centrifuge at 4 °C and 2000 rpm for 5 minutes, and discard the supernatant. Take 5 mL of red blood cell lysate and resuspend spleen cells. Use a pipette to sufficiently disperse the cells, incubate at room temperature (on ice) for 5 minutes, then centrifuge at 4 °C and 2000 rpm for 5 minutes. Discard the supernatant. Add 1000 µL PBS buffer to resuspend the cells and transfer them to a 1.5 mL centrifuge tube for later use. Mouse lymphocytes were seeded into a 96 well microplate at a density of 1.5 × 10⁶/mL. Anti mouse CD3 antibody (Thermo Fisher Scientific (China) Co., Ltd., Cat# 16-0031-85) was added at a concentration of 1 µg/mL, and at the same time, pre-prepared mouse red blood cells coupled with 3H3 antibody were also added to co-stimulate the spleen cells. Alternatively, anti-mouse 4-1BB antibody (10 nM 3H3 protein, 100 nM LOB protein) was added as a control. After 48 hours, the cells were collected and treated with anti-CD8 (Wuhan Elabscience Biotechnology Co., Ltd., Cat# E-AB-F1104J, diluted to 1:50) and anti-CD4 (Wuhan Elabscience Biotechnology Co., Ltd., Cat# E-AB-F-1104J, diluted to 1:200), and NK1.1 (Wuhan Elabscience Biotechnology Co., Ltd., Cat# E-AB-F-1104J, diluted to 1:200) respectively, stained and flow cytometry analyses were performed.

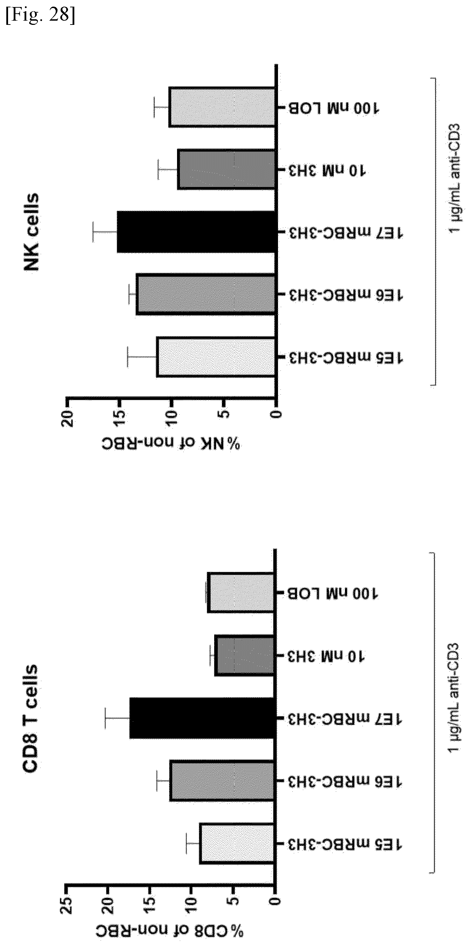

The left side of Figure 28 shows the proportion of CD8⁺T cells detected by flow cytometry after co-stimulation with 1µg/mL anti-mouse CD3 antibody and anti-mouse 4-1BB antibodies under 5 different conditions: the proportion of CD8⁺ T cells produced by co-stimulation with 1 × 10⁵ 3H3 coupled red blood cells (1E5 mRBC-3H3) was 9.1%; the proportion of CD8⁺ T cells produced by co-stimulation with 1 × 10⁶ 3H3 coupled red blood cells (1E6 mRBC-3H3) was 12.6%; the proportion of CD8⁺ T cells produced by co-stimulation with 1 × 10⁷ 3H3 coupled red blood cells (1E7 mRBC-3H3) was 17.5%; the proportion of CD8⁺T cells produced by co-stimulation with 10 nM 3H3 protein was 7.2%; the proportion of CD8⁺T cells produced by co-stimulation with 100 nM LOB protein was 8.1%. The results showed that the proportion of CD8⁺ T cells stimulated by anti-mouse 4-1BB antibody (10 nM 3H3 protein or 100 nM LOB protein) was only 7% - 8%, while the proportion of CD8⁺ T cells stimulated by mouse red blood cells coupled with the anti-mouse 4-1BB antibodies was higher (9% - 17.5%), and increased with the increase of red blood cell number.

The right side of Figure 28 shows the proportion of NK cells detected by flow cytometry after co-stimulation with 1 µg/mL anti-mouse CD3 antibody and anti-mouse 4-1BB antibodies under 5 different conditions: the proportion of NK cells produced by co-stimulation with 1 × 10⁵ 3H3 coupled red blood cells (1E5 mRBC-3H3) was 11.5%; the proportion of NK cells produced by co-stimulation with 1 × 10⁶ 3H3 coupled red blood cells (1E6 mRBC-3H3) was 13.5%; the proportion of NK cells produced by co-stimulation with 1 × 10⁷ 3H3 coupled red blood cells (1E7 mRBC-3H3) was 15.3%; the proportion of NK cells produced by co-stimulation with 10 nM 3H3 protein was 9.5%; the proportion of NK cells produced after co-stimulation with 100 nM LOB protein was 10.3%. The results showed that the proportion of NK cells stimulated by anti-mouse 4-1BB antibody (10 nM 3H3 protein or 100 nM LOB protein) was only about 10%, while the proportion of NK cells stimulated by mouse red blood cells coupled with the anti-mouse 4-1BB antibodies was higher (11.5% - 15.3%), and increased with the increase of red blood cell number.

In summary, mouse red blood cells coupled with anti-4-1BB antibodies can effectively stimulate the proliferation of CD8⁺ T cells and NK cells.

In order to further investigate the effect on CD8⁺ T lymphocytes, CD8⁺ T lymphocytes were isolated and purified. Prepared mouse spleen single cells were resuspended in MojoSort^{™} Buffer (BioLegend, CAT #. 480007), and after counting, 10⁷ cells were separated, and 10 µL Biotin Antibody Cocktail was added. After incubation on ice for 15 minutes, 10 µL Streptavidin Nanobeads were added and mixed thoroughly. After incubation on ice for 15 minutes, the cells were placed on a magnetic rack and the supernatant was collected. The proportion of CD8⁺ T in the supernatant exceeded 95%. CD8⁺T was seeded into a 96 well microplate at a density of 10⁶/mL, and 2 µg/mL of anti-mouse CD3 antibody was added along with prepared mouse red blood cells coupled with 3H3 antibody. After incubation for 48 hours, the culture supernatant was collected and the interferon γ concentration in the supernatant was detected using a mouse interferon gamma ELISA kit (Beijing solarbio Technology Co., Ltd.,Cat#. SEKM-0031).

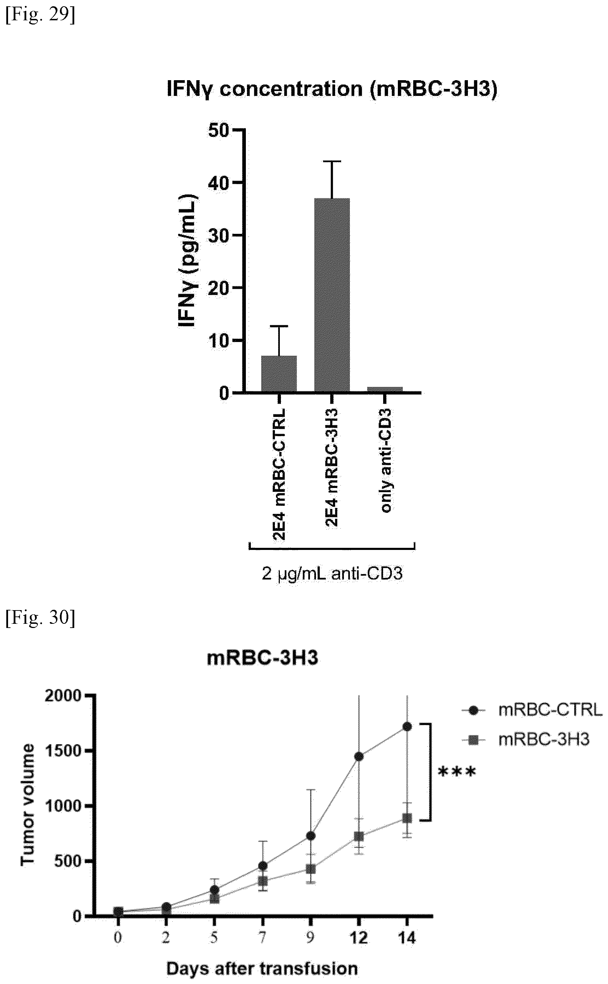

Figure 29 shows that under the stimulation of 2 µg/mL anti-mouse CD3 antibody, 2 × 10⁴ uncoupled red blood cells (referred to as 2E4 mRBC-CTRL, left) or 2 × 10⁴ 3H3 coupled red blood cells (referred to as 2E4 mRBC-3H3, middle) were further added, and non-added red blood cells were used as a blank control (referred to as only anti-CD3, right). ELISA detection was used to determine the concentration of interferon γ secreted by CD8⁺ T cells under the stimulation of the three groups: the concentration of interferon γ produced by the 2E4 mRBC-CTRL group was 7 pg/mL, the concentration of interferon γ produced by the 2E4 mRBC-3H3 group was 37 pg/mL, and the concentration of interferon γ produced by the only anti-CD3 group was 1 pg/mL. The results showed that mouse red blood cells coupled with anti-4-1BB antibodies significantly increased CD8⁺T secretion of interferon γ (Figure 29). Interferon γ is mainly produced by activated T cells and NK cells, and has antiviral, immune regulatory, and anti-tumor properties.

*In vitro* experiments have shown that mouse red blood cells coupled with anti-4-1BB antibodies can effectively activate CD8⁺ T and NK cells as immune activators

### Example 12. Mouse red blood cells coupled with anti-mouse 4-1BB antibody as immunomodulatory drugs for the treatment of CT26 colon cancer tumor model

Balb/C mice were purchased by InnoModels Biotechnology (Beijing) Co., Ltd. and raised at InnoModels Biotechnology. Approximately 2*10⁵ CT-26 cells (mouse colon cancer cell line, provided by InnoModels Biotech) were subcutaneously injected into the right scapula of 5-12 week old mice. The tumor volume was measured along three orthogonal axes (a, b, and c) and calculation as tumor volume = abc/2. Mouse red blood cells were coupled with anti-mouse 4-1BB antibody 3H3 according to the method described in Example 3 or Example 4. After tumor establishment (about 9-12 days), mice were infused with 3H3 antibody coupled red blood cells or red blood cells without the coupled antibody via tail vein once a week, at a dose of 5 × 10⁸ each time, for 3 weeks. After infused red blood cells, the tumor volume and body weight of the mice were measured three times a week, and the general condition of the mice was recorded.

Figure 30 shows the trend of tumor volume changes in the mice from the first infusion of 3H3 antibody coupled red blood cells or red blood cells without conjugated-antibody, continuously monitored for 14 days. On the 14th day, the average tumor volume of the mice infused with the antibody uncoupled red blood cells reached 1720.8 mm³, and the average tumor volume of the mice infused with the antibody 3H3 coupled red blood cells was 890.3 mm³, which was 51.7% of the tumor volume of the mice infused with the antibody uncoupled red blood cells. After being infused with the red blood cells coupled with 3H3 antibody, the tumor of the mice was significantly inhibited compared to the red blood cells infused with the antibody uncoupled red blood cells.

*In vivo* pharmacodynamics tests have shown that the mouse red blood cells coupled with the anti-mouse 4-1BB antibody can be used as an immunomodulatory drug to treat CT26 colon cancer.

## Claims

1. A method for coupling a chemical molecule or a biomacromolecule to the surface of a mature red blood cell, wherein the method comprises the following steps:
(1) coupling the chemical molecule or the biomacromolecule with a GDP-fucose derivative;
(2) covalently coupling the chemical molecule or the biomacromolecule obtained from step (1) with a polysaccharide on the membrane surface of the red blood cell through a glycosidic bond using an enzymatic reaction mediated by fucosyltransferase.

2. The method according to claim 1, wherein the chemical molecule is selected from therapy molecules or fluorophores.

3. The method according to claim 1, wherein the biomacromolecule is selected from polynucleotide, polypeptide, antibody.

4. The method according to claim 3, wherein the antibody is selected from monoclonal antibody, single chain antibody, bi-specific antibody, nano antibody.

5. The method according to claim 4, wherein the antibody is anti-4-1BB activating antibody.

6. The method according to claim 3, wherein the biomacromolecule is selected from interleukin-15 isomer or interleukin-12.

7. The method according to claim 1, wherein the mature red blood cell is obtained from peripheral blood without serum and other blood cells, red blood cell suspension without free protein molecules, fresh red blood cell suspension or red blood cell suspension stored under a suitable condition within 30 days.

8. The method according to claim 1, wherein the fucosyltransferase is from a human being or from Helicobacter pylori.

9. The method according to claim 8, wherein the GDP-fucose derivative is Guanosine 5'-diphosphate-fucose-triazole polyethylene glycol-methyl tetrazine.

10. The method according to claim 9, wherein the reaction conditions of the enzymatic reaction mediated by fucosyltransferase are: 5× 10⁷/mL-5 × 10⁹/mL for red blood cell density; 40 µg/mL-300 µg/mL of fucosyltransferase; 60 µg/mL-120 µg/mL of the antibody or Fc fusion protein as the macromolecular drug; 4°C-7°C for reaction temperature; 20 minutes-16 hours of reaction time.

11. The method according to claim 11 wherein the enzymatic reaction mediated by fucosyltransferase is carried out in a reaction system of 150mM NaCl, 2.7mM KCl, 44mM glucose, pH 5.9-6.3, but without Mg²⁺.

12. An engineered red blood cell, wherein the engineered red blood cell is prepared according to the method in any one of claims 1-10.

13. The engineered red blood cell according to claim 12 for use as a therapeutic drug for a tumor disease.

14. The use according to claim 13, wherein the tumor disease is colon cancer cells or melanoma.
